# EUROPÄISCHE PATENTSCHRIFT

(11) **EP 0 967 209 B1**
(45) Veröffentlichungstag und Bekanntmachung des Hinweises auf die Patenterteilung: **08.01.2003**
(21) Anmeldenummer: 99105458.6
(22) Anmeldetag: 17.03.1999
(51) Int. Cl.: C07D 239/54, C08K 5/3462

(54) **Neue NH2-modifizierte 6-Aminouracile als Stabilisatoren für halogenhaltige Polymere**
NH2-modified 6-aminouracils as stabilisers for halogen-containing polymers
6-aminouraciles modifiés sur le NH2, comme stabilisateurs pour des polymères halogènes

(30) Priorität: 26.06.1998 CH 137098
(43) Veröffentlichungstag der Anmeldung: 29.12.1999
(73) Patentinhaber: Crompton Vinyl Additives GmbH, 68623 Lampertheim (DE)
(72) Erfinder: Wehner, Wolfgang Dr., 64673 Zwingenberg (DE); Friedrich, Hans-Helmut, 64686 Lautertal-Gadernheim (DE); Drewes, Rolf Dr., 79395 Neuenburg (DE)

(56) Entgegenhaltungen:
- EP-A- 0 065 934
- DE-A- 19 741 778
- FR-A- 2 232 320

## Beschreibung

Die Erfindung betrifft 1,3-disubstituierte 6-Aminouracile der unten dargestellten Formel I zum Stabilisieren chlorhaltiger Polymere, insbesondere PVC.

PVC kann durch eine Reihe von Zusatzstoffen stabilisiert werden. Verbindungen des Bleis, Bariums und Cadmiums sind dafür besonders gut geeignet, sind jedoch heute aus ökologischen Gründen oder wegen ihres Schwermetallgehalts umstritten (vgl. "Kunststoffadditive", R. Gächter/H. Müller, Carl Hanser Verlag, 3. Aufl., 1989, Seiten 303-311, und "Kunststoff Handbuch PVC", Band 2/1, W. Becker/D. Braun, Carl Hanser Verlag, 2. Aufl., 1985, Seiten 531 - 538; sowie Kirk-Othmer: "Encyclopedia of Chemical Technology", 4^{th} Ed., 1994, Vol. 12, Heat Stabilizers, S. 1071 - 1091). Man sucht daher weiter nach wirksamen Stabilisatoren und Stabilisatorkombinationen, welche frei von Blei, Barium und Cadmium sind.

Die EP-A-0 065 934 schlägt Aminouracile zur Stabilisierung von thermoplastischen Formmassen, insbesondere Vinylchloridpolymeren, vor. Als bevorzugte Aminouracile benennt dieses Dokument 1,3-Di-n-butyl-6-aminouracil und 1,3-Diäthyl-6-aminouracil.

Aus der DE 197 41 778 A1 sind Zusammensetzungen bekannt, die Hart- oder Halbhart-PVC mit einem Weichmacher-Anteil von bis zu 20% und bestimmte N,N-Dimethyl-6-aminouracile enthalten.

1,3-disubstituierte Aminouracile sind bereits in US 3,436,362, US 4,656,209, US 4,352,903 und EP-A-0 768 336 beschrieben worden, und können nach bekannten Methoden in einem (oder mehreren) Verfahrensschritt(en) hergestellt werden.

Es wurde nun gefunden, dass die 1,3-disubstituierte 6-Aminouracile der allgemeinen Formel I 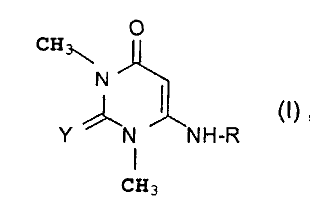 wobei
- Y: Sauerstoff oder Schwefel ist, und
- R: mit -OH substituiertes Phenyl oder mit -OH und 1 bis 3 C₁-C₄-Alkyl oder mit C₁-C₄-Alkoxy substituiertes Phenyl darstellt,
sich besonders gut für die Stabilisierung von chlorhaltigen Polymeren wie z. B. PVC eignen.

Für Verbindungen der Formel I gilt:
C₁-C₄-Alkyl bedeutet z. B. Methyl, Ethyl, n-Propyl, iso-Propyl, n-, i-, sec- oder t-Butyl.

C₁-C₄-Alkoxy bedeutet beispielsweise Methoxy, Ethoxy, Propoxy, und n-, i- und tert.-Butoxy.
Bevorzugt sind Methoxy und Ethoxy.

Die Verbindungen der Formel I sind zur Erzielung der Stabilisierung im chlorhaltigen Polymer zweckmäßig zu 0,01 bis 10 Gew.-%, vorzugsweise zu 0,05 bis 5 Gew.-%, insbesondere zu 0,1 bis 3 Gew.-% zu verwenden.

Weiterhin können Kombinationenen von Verbindungen der allgemeinen Formel I mit anderen üblichen Additiven bzw. Stabilisatoren eingesetzt werden, beispielsweise mit Polyolen und Disaccharidalkoholen und/oder Perchloratverbindungen und/oder Glycidylverbindungen und/oder zeolithischen Verbindungen und/oder Schichtgitterverbindungen (Hydrotalcite) sowie z. B. Lichtschutzmittel.
Beispiele für solche zusätzlichen Komponenten sind nachfolgend aufgeführt und erläutert.

### Polyole und Disaccharidalkohole

Als Verbindungen dieses Typs kommen beispielsweise in Betracht:
Pentaerythrit, Dipentaerythrit, Tripentaerythrit, Trimethylolethan, Bistrimethylolpropan, Inosit (Cyclite), Polyvinylalkohol, Bistrimethylolethan, Trimethylolpropan, Sorbit (Hexite), Maltit, Isomaltit, Cellobiit, Lactit, Lycasin, Mannit, Lactose, Leucrose, Tris-(hydroxyethyl)-isocyanurat, Tris-(hydroxypropyl)-isocyanurat, Palatinit, Tetramethylolcyclohexanol, Tetramethylolcyclopentanol, Tetramethylolcyclopyranol, Xylit, Arabinit (Pentite), Tetrite, Glycerin, Diglycerin, Polyglycerin, Thiodiglycerin oder 1-0-a-D-Glycopyranosyl-D-mannit-dihydrat. Bevorzugt sind davon die Disaccharidalkohole.
Verwendung finden können auch Polyolsirupe, wie Sorbit-, Mannit- und Maltitsirup.
Die Polyole können in einer Menge von beispielsweise 0,01 bis 20, zweckmäßig von
0,1 bis 20 und insbesondere von 0,1 bis 10 Gew.-Teilen bezogen auf 100 Gew.-Teile PVC, angewandt werden.

### Perchlorat-Verbindungen

Beispiele sind diejenigen der Formel M(ClO₄)_{n,} wobei M für Li, Na, K, Mg, Ca, Sr, Ba, Zn, Al, La oder Ce steht. Der Index n ist entsprechend der Wertigkeit von M 1, 2 oder 3. Die Perchloratsalze können mit Alkoholen (Polyolen,Cyclodextrinen), oder Ätheralkoholen bzw. Esteralkoholen komplexiert oder gelöst sein. Zu den Esteralkoholen sind auch die Polyolpartialester zu zählen. Bei mehrwertigen Alkoholen oder Polyolen kommen auch deren Dimere, Trimere, Oligomere und Polymere in Frage, wie Di-, Tri-, Tetra- und Polyglycole, sowie Di-, Tri- und Tetrapentaerythrit oder Polyvinylalkohol in verschiedenen Polymerisationsgraden.
Als weitere Lösungsmittel kommen Phosphatester sowie cyclische und acyclische Kohlensäureester in Frage.
Die Perchloratsalze können dabei in verschiedenen gängigen Darreichungsformen eingesetzt werden; z. B als Salz oder Lösung in Wasser oder einem organischen Solvens als solches, bzw. aufgezogen auf ein Trägermaterial wie PVC, Ca-Silikat, Zeolithe oder Hydrotalcite, oder eingebunden durch chemische Reaktion in einen Hydrotalcit oder eine andere Schichtgitterverbindung. Als Polyolpartialether sind Glycerinmonoether und Glycerinmonothioether bevorzugt.
Weitere Ausführungsformen werden beschrieben in EP 0 394 547, EP 0 457 471 und WO 94/24200.
Die Perchlorate können in einer Menge von beispielsweise 0,001 bis 5, zweckmäßig 0,01 bis 3, besonders bevorzugt 0,01 bis 2 Gew.-Teilen, bezogen auf 100 Gew.-Teile PVC, angewandt werden.

### Glycidylverbindungen

Sie enthalten die Glycidylgruppe 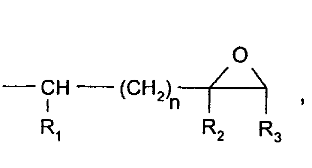 wobei diese direkt an Kohlenstoff, Sauerstoff-, Stickstoff- oder Schwefelatome gebunden ist, und worin entweder R₁ und R₃ beide Wasserstoff sind, R₂ Wasserstoff oder Methyl und n = 0 ist, oder worin R₁ und R₃ zusammen -CH₂-CH₂- oder -CH₂-CH₂-CH₂- bedeuten, R₂ dann Wasserstoff und
n = 0 oder 1 ist.
I) Glycidyl- und β-Methylglycidylester erhältlich durch Umsetzung einer Verbindung mit mindestens einer Carboxylgruppe im Molekül und Epichlorhydrin bzw. Glycerindichlorhydrin bzw. β-Methyl-epichlorhydrin. Die Umsetzung erfolgt zweckmäßig in Gegenwart von Basen.

Als Verbindungen mit mindestens einer Carboxylgruppe im Molekül können aliphatische Carbonsäuren verwandt werden. Beispiele für diese Carbonsäuren sind Glutarsäure, Adipinsäure, Pimelinsäure, Korksäure, Azelainsäure, Sebazinsäure oder dimerisierte bzw. trimerisierte Linolsäure, Acryl- und Methacrylsäure, Capron-, Capryl-, Laurin-, Myristin-, Palmitin-, Stearin- und Pelargonsäure, sowie die bei den organischen Zinkverbindungen erwähnten Säuren.
Es können aber auch cycloaliphatische Carbonsäuren eingesetzt werden, wie beispielsweise Cyclohexancarbonsäure, Tetrahydrophthalsäure, 4-Methyltetrahydrophthalsäure, Hexahydrophthalsäure oder 4-Methylhexahydrophthalsäure. Weiterhin können aromatische Carbonsäuren Verwendung finden, wie beispielsweise Benzoesäure, Phthalsäure, Isophthalsäure, Trimellithsäure oder Pyromellithsäure.
Ebenfalls können auch carboxylterminierte Addukte, z. B. von Trimellithsäure und Polyolen, wie beispielsweise Glycerin oder 2,2-Bis-(4-hydroxycyclohexyl)-propan verwandt werden.
Weitere im Rahmen dieser Erfindung verwendbare Epoxidverbindungen finden sich in der EP 0506 617.
II) Glycidyl- oder (β-Methylglycidyl)-ether erhältlich durch Umsetzung einer Verbindung mit mindestens einer freien alkoholischen Hydroxygruppe und/oder phenolischen Hydroxygruppe und einem geeignet substituierten Epichlorhydrin unter alkalischen Bedingungen, oder in Anwesenheit eines sauren Katalysators und anschließender Alkalibehandlung.

Ether dieses Typs leiten sich beispielsweise ab von acyclischen Alkoholen, wie Ethylenglykol, Diethylenglykol und höheren Poly-(oxyethylen)-glykolen, Propan-1,2-diol, oder Poly-(oxypropylen)-glykolen, Propan-1,3-diol, Butan-1,4-diol, Poly-(oxytetramethylen)-glykolen, Pentan-1,5-diol, Hexan-1,6-diol, Hexan-2,4,6-triol, Glycerin, 1,1,1-Trimethylolpropan, Bistrimethylolpropan, Pentaerythrit, Sorbit, sowie von Polyepichlorhydrinen, Butanol, Amylalkohol, Pentanol, sowie von monofunktionellen Alkoholen wie Isooctanol, 2-Ethylhexanol, Isodecanol sowie C₇-C₉-Alkanol- und C₉-C₁₁-Alkanolgemischen.
Sie leiten sich aber auch beispielsweise ab von cycloaliphatischen Alkoholen wie 1,3- oder 1,4-Dihydroxycyclohexan, Bis-(4-hydroxycyclohexyl)-methan, 2,2-Bis-(4-hydroxycyclohexyl)-propan oder 1,1-Bis-(hydroxymethyl)-cyclohex-3-en oder sie besitzen aromatische Kerne wie N,N-Bis-(2-hydroxyethyl)-anilin oder p,p'-Bis-(2-hydroxyethylamino)-diphenylmethan.
Die Epoxidverbindungen können sich auch von einkernigen Phenolen ableiten, wie beispielsweise von Phenol, Resorcin oder Hydrochinon; oder sie basieren auf mehrkernigen Phenolen wie beispielsweise auf Bis-(4-hydroxyphenyl)-methan, 2,2-Bis-(4-hydroxyphenyl)-propan, 2,2-Bis-(3,5-dibrom-4-hydroxyphenyl)-propan, 4,4'-Dihydroxydiphenylsulfon oder auf unter sauren Bedingungen erhaltene Kondensationsprodukte von Phenolen mit Formaldehyd wie Phenol-Novolake.
Weitere mögliche endständige Epoxide sind beispielsweise: Glycidyl-1-naphthylether, Glycidyl-2-phenylphenylether, 2-Biphenylglycidylether, N-(2,3-epoxypropyl)-phthalimid und 2,3-Epoxypropyl-4-methoxyphenylether.
III) (N-Glycidyl)-Verbindungen erhältlich durch Dehydrochlorierung der Reaktionsprodukte von Epichlorhydrin mit Aminen, die mindestens ein Aminowasserstoffatom enthalten. Bei diesen Aminen handelt es sich zum Beispiel um Anilin, N-Methylanilin, Toluidin, n-Butylamin, Bis-(4-aminophenyl)-methan, m-Xylylendiamin oder Bis-(4-methylaminophenyl)-methan, aber auch N,N,O-Triglycidyl-m-aminophenol oder N,N,O-Triglycidyl-p-aminophenol.

Zu den (N-Glycidyl)-Verbindungen zählen aber auch N,N'-Di-, N,N',N"-Tri- und N,N',N",N'''-Tetraglycidylderivate von Cycloalkylenharnstoffen, wie Ethylenharnstoff oder 1,3-Propylenharnstoff, und N,N'-Diglycidylderivate von Hydantoinen, wie von 5,5-Dimethylhydantoin oder Glykoluril und Triglycidylisocyanurat.
IV) S-Glycidyl-Verbindungen, wie beispielsweise Di-S-glycidylderivate, die sich von Dithiolen, wie beispielsweise Ethan-1,2-dithiol oder Bis-(4-mercaptomethylphenyl)-ether ableiten.

V) Epoxidverbindungen mit einem Rest der obigen Formel, worin R₁ und R₃ zusammen -CH₂-CH₂- bedeuten und n 0 ist, sind Bis-(2,3-epoxycyclopentyl)-ether, 2,3-Epoxycyclopentylglycidylether oder 1,2-Bis-(2,3-epoxycyclopentyloxy)-ethan. Ein Epoxidharz mit einem Rest der obigen Formel, worin R₁ und R₃ zusammen -CH₂-CH₂- sind und n 1 bedeutet, ist beispielsweise 3,4-Epoxy-6-methyl-cyclohexancarbonsäure-(3',4'-epoxy-6'-methyl-cyclohexyl)-methylester.

Geeignete endständige Epoxide sind beispielsweise:
a) flüssige Bisphenol-A-diglycidylether wie Araldit®GY 240, Araldit®GY 250, Araldit®GY 260, Araldit®GY 266, Araldit®GY 2600, Araldit®MY 790;
b) feste Bisphenol-A-diglycidylether wie Araldit®GT 6071, Araldit®GT 7071, Araldit®GT 7072, Araldit®GT 6063, Araldit®GT 7203, Araldit®GT 6064, Araldit®GT 7304, Araldit®GT 7004, Araldit®GT 6084, Araldit®GT 1999, Araldit®GT 7077, Araldit®GT 6097, Araldit®GT 7097, Araldit®GT 7008, Araldit®GT 6099, Araldit®GT 6608, Araldit®GT 6609, Araldit®GT 6610;
c) flüssige Bisphenol-F-diglycidylether wie Araldit®GY 281, Araldit®PY 302, Araldit®PY 306;
d) feste Polyglycidylether von Tetraphenylethan wie CG Epoxy Resin®0163;
e) feste und flüssige Polyglycidylether von Phenolformaldehyd Novolak wie EPN 1138, EPN 1139, GY 1180, PY 307;
f) feste und flüssige Polyglycidylether von o-Cresolformaldehyd Novolak wie ECN 1235, ECN 1273, ECN 1280, ECN 1299;
g) flüssige Glycidylether von Alkoholen wie Shell® Glycidylether 162, Araldit®DY 0390, Araldit®DY 0391;
h) flüssige Glycidylether von Carbonsäuren wie Shell®Cardura E Terephthalsäureester, Tri mellithsäureester, Araldit®PY 284;
i) feste heterocyclische Epoxidharze (Triglycidylisocyanurat) wie Araldit® PT 810;
j) flüssige cycloaliphatische Epoxidharze wie Araldit®CY 179;
k) flüssige N,N,O-Triglycidylether von p-Aminophenol wie Araldit®MY 0510;
l) Tetraglycidyl-4-4'-methylenbenzamin oder N,N,N',N'-Tetraglycidyldiaminophenylmethan wie Araldit®MY 720, Araldit®MY 721.

Vorzugsweise finden Epoxidverbindungen mit zwei funktionellen Gruppen Verwendung. Es können aber auch prinzipiell Epoxidverbindungen mit einer, drei oder mehr funktionellen Gruppen eingesetzt werden.
Vorwiegend werden Epoxidverbindungen, vor allem Diglycidylverbindungen, mit aromatischen Gruppen eingesetzt.
Gegebenenfalls kann auch ein Gemisch verschiedener Epoxidverbindungen eingesetzt werden.
Besonders bevorzugt sind als endständige Epoxidverbindungen Diglycidylether auf der Basis von Bisphenolen, wie beispielsweise von 2,2-Bis-(4-hydroxyphenyl)-propan (Bisphenol-A), Bis-(4-hydroxyphenyl)-methan oder Mischungen von Bis-(ortho/para-hydroxyphenyl)-methan (Bisphenol-F).
Die endständigen Epoxidverbindungen können in einer Menge von vorzugsweise mindestens 0,1 Teil, beispielsweise 0,1 bis 50, zweckmäßig 1 bis 30 und insbesondere 1 bis 25 Gew.-Teilen, bezogen auf 100 Gew.-Teile PVC, eingesetzt werden.

### Hydrotalcite

Die chemische Zusammensetzung dieser Verbindungen ist dem Fachmann bekannt,
z. B. aus den Patentschriften DE 3 843 581, US 4,000,100, EP 0 062 813 und WO 93/20135.
Verbindungen aus der Reihe der Hydrotalcite können durch die folgende allgemeine Formel

M²⁺ ₁₋ₓ M³⁺ ₓ(OH)₂ (A^{b-})_{x/b} • d H₂O

beschrieben werden,
wobei
M²⁺ = eines oder mehrere der Metalle aus der Gruppe Mg, Ca, Sr, Zn oder Sn ist,
M³⁺ = Al, oder B ist,
- Aⁿ: ein Anion mit der Valenz n darstellt,
b eine Zahl von 1 - 2 ist,
0 < x <0,5 ist,
- m: eine Zahl von 0 - 20 ist.
Bevorzugt ist
- Aⁿ: = OH⁻, ClO₄⁻, HCO₃⁻, CH₃COO⁻, C₆H₅COO⁻, CO₃²⁻, (CHOHCOO)₂²⁻, (CH₂COO)₂²⁻, CH₃CHOHCOO⁻, HPO₃⁻ oder HPO₄²⁻
darstellt;
Beispiele für Hydrotalcite sind
Al₂O₃.6MgO.CO₂.12H₂O (i), Mg_{4,5}Al₂(OH)₁₃.CO₃.3,5H₂O (ii), 4MgO.Al₂O₃.CO₂.9H₂O (iii), 4MgO.Al₂O₃.CO₂.6H₂O,
ZnO.3MgO.Al₂O₃.CO₂.8-9H₂O und ZnO.3MgO.Al₂O₃.CO₂.5-6H₂O.
Ganz besonders bevorzugt sind die Typen i, ii und iii.

### Zeolithe (Alkali bzw. Erdalkalialumosilikate)

Sie können durch die folgende allgemeine Formel

M_{x/n}[(AlO₂)ₓ(SiO₂)_{y}].wH₂O

beschrieben werden,
worin n die Ladung des Kations M;
M ein Element der ersten oder zweiten Hauptgruppe, wie Li, Na, K, Mg, Ca, Sr oder Ba;
y : x eine Zahl von 0,8 bis 15, bevorzugt von 0,8 bis 1,2; und
w eine Zahl von 0 bis 300, bevorzugt von 0,5 bis 30, ist.
Beispiele für Zeolithe sind Natriumalumosilikate der Formeln Na₁₂Al₁₂Si₁₂O₄₈ . 27 H₂O [Zeolith A], Na₆Al₆Si₆O₂₄ . 2 NaX . 7,5 H₂O, X= OH, Halogen, ClO₄ [Sodalith]; Na₆Al₆Si₃₀O₇₂ . 24 H₂O; Na₈Al₈Si₄₀O₉₆ . 24 H₂O; Na₁₆Al₁₆Si₂₄O₈₀ . 16 H₂O; Na₁₆Al₁₆Si₃₂O₉₆. 16 H₂O; Na₅₆Al₅₆Si₁₃₆O₃₈₄ . 250 H₂O [Zeolith Y], Na₈₆Al₈₆Si₁₀₆O₃₈₄ . 264 H₂O [Zeolith X];
oder die durch teilweisen bzw. vollständigen Austausch der Na-Atome durch Li-, K-, Mg-, Ca-, Sr- oder Zn-Atome darstellbaren Zeolithe wie (Na,K)₁₀Al₁₀Si₂₂O₆₄ . 20 H₂O ; Ca_{4,5}Na₃[(AlO₂)₁₂(SiO₂)₁₂]. 30 H₂O; K₉Na₃[(AlO₂)₁₂(SiO₂)₁₂] . 27 *H*_{*2*}*O*,
Bevorzugte Zeolithe entsprechen den Formeln
Na₁₂Al₁₂Si₁₂O₄₈.27 H₂O [Zeolith A],
Na₆Al₆Si₆O₂₄ . 2NaX . 7,5 H₂O, X = OH, Cl, ClO₄, 1/2CO₃ [Sodalith]
Na₆Al₆Si₃₀O₇₂ . 24 H₂O,
Na₈Al₈Si₄₀O₉₆ . 24 H₂O,
Na₁₆Al₁₆Si₂₄O₈₀ . 16 H₂O,
Na₁₆Al₁₆Si₃₂O₉₆ . 16 H₂O,
Na₅₆Al₅₆Si₁₃₆O₃₈₄ . 250 H₂O [Zeolith Y],
Na₈₆Al₈₆Si₁₀₆O₃₈₄ . 264 H₂O [Zeolith X]
und solche X- und Y-Zeolithe mit einem Al/ Si-Verhältnis von ca. 1:1.
oder die durch teilweisen bzw. vollständigen Austausch der Na-Atome durch Li-, K-, Mg-, Ca-, Sr-, Ba- oder Zn-Atome darstellbaren Zeolithe wie
(Na,K)₁₀Al₁₀Si₂₂O₆₄ . 20 H₂O .
Ca_{4,5}Na₃[(AlO₂)₁₂(SiO₂)₁₂] . 30 H₂O
K₉Na₃[(AlO₂)₁₂(SiO₂)₁₂] . 27 H₂O
Die angeführten Zeolithe können auch wasserärmer bzw. wasserfrei sein. Weitere geeignete Zeolithe sind:
Na₂O·Al₂O₃·(2 bis 5) SiO₂·(3,5 bis 10) H₂O [Zeolith P]
Na₂O·Al₂O₃·2 SiO₂·(3.5-10)H₂O (Zeolith MAP)
oder die durch teilweisen bzw. vollständigen Austausch der Na-Atome durch Li-, K- oder H-Atome darstellbaren Zeolithe wie
(Li,Na,K,H)₁₀Al₁₀Si₂₂O₆₄ . 20 H₂O.
K₉Na₃[(AlO₂)₁₂(SiO₂)₁₂] . 27 H₂O
K₄Al₄Si₄O₁₆·6H₂O [Zeolith K-F]
Na₈Al₈Si₄₀O₉₆.24 H₂O [Zeolith D], wie in Barrer et al., J. Chem. Soc. **1952,** 1561 - 71, und in US 2,950,952 beschrieben;
Ferner kommen folgende Zeolithe in Frage:
K-Offretit, wie in EP-A-400,961 beschrieben;
Zeolith R, wie in GB 841,812 beschrieben;
Zeolith LZ-217, wie in US 4,503,023 beschrieben;
Ca-freier Zeolith LZ-218, wie in US 4,333,859 beschrieben;
Zeolith T, Zeolith LZ-220, wie in US 4,503,023 beschrieben;
Na₃K₆Al₉Si₂₇O₇₂.21 H₂O [Zeolith L];
Zeolith LZ-211, wie in US 4,503,023 beschrieben;
Zeolith LZ-212, wie in US 4,503,023 beschrieben;
Zeolith O, Zeolith LZ-217, wie in US 4,503,023 beschrieben;
Zeolith LZ-219, wie in US 4,503,023 beschrieben;
Zeolith Rho, Zeolith LZ-214, wie in US 4,503,023 beschrieben;
Zeolith ZK-19, wie in Am. Mineral. **54** 1607 (1969) beschrieben;
Zeolith W (K-M), wie in Barrer et al., J. Chem. Soc. **1956**, 2882, beschrieben;
Na₃₀Al₃₀Si₆₆O₁₉₂. 98 H₂O [Zeolith ZK-5, Zeolith Q]
Besonders bevorzugt werden Zeolith P-Typen der obigen Formel verwandt,
worin x 2 bis 5 und y 3.5 bis 10 sind, ganz besonders bevorzugt Zeolith MAP der genannten Formel, worin x 2 und y 3.5 bis 10 sind. Insbesondere handelt es sich um Zeolith Na-P, d. h. M steht für Na. Dieser Zeolith tritt im allgemeinen in den Varianten Na-P-1, NaP-2 und Na-P-3 auf, die sich durch ihre kubische, tetragonale oder orthorhombische Struktur unterscheiden (R. M. Barrer, B. M. Munday, J.Chem.Soc. A **1971**, 2909- 14). In der ebengenannten Literatur ist auch die Herstellung von Zeolith P-1 und P-2 beschrieben. Zeolith P-3 ist danach sehr selten und daher kaum von praktischem Interesse. Die Struktur des Zeolithen P-1 entspricht der aus dem obengenannten Atlas of Zeolite Structures bekannten Gismonditstruktur. In neuerer Literatur
(EP-A-384 070) wird zwischen kubischem (Zeolith B oder P_{c}) und tetragonalem (Zeolith P₁) Zeolithen vom P-Typ unterschieden. Dort werden auch neuere Zeolithen des P-Typs mit Si:Al Verhältnissen unter 1,07:1 genannt. Hierbei handelt es sich um Zeolithe mit der Bezeichnung MAP oder MA-P für "Maximum Aluminium P". Je nach Herstellungsverfahren kann Zeolith P geringe Anteile anderer Zeolithe enthalten. Sehr reiner Zeolith P ist in WO 94/26662 beschrieben worden.
Im Rahmen der Erfindung lassen sich auch solche feinteiligen, wasserunlöslichen Natriumalumosilikate verwenden, die in Gegenwart von wasserlöslichen anorganischen oder organischen Dispergiermitteln gefällt und kristallisiert wurden. Diese können in beliebiger Weise vor oder während der Fällung bzw. Kristallisation in das Reaktionsgemisch eingebracht werden.
Ganz besonders bevorzugt sind Na-Zeolith A und Na-Zeolith P.
Die Hydrotalcite und/oder Zeolithe können in Mengen von beispielsweise 0,1 bis 20, zweckmäßig 0,1 bis 10 und insbesondere 0,1 bis 5 Gew.-Teilen, bezogen auf
100 Gew.-Teile halogenhaltiges Polymer angewandt werden.

Die erfindungsgemässen Zusammensetzungen können auch mit weiteren üblichen Zusatzstoffen versetzt sein, wie etwa Stabilisierungs-, Hilfs- und Verarbeitungsmitteln, z. B. Alkali- und Erdalkaliverbindungen, Gleitmitteln, Weichmachern, Pigmenten, Füllstoffen, Phosphiten, Thiophosphiten und Thiophosphaten, Mercaptocarbonsäureestern, epoxidierten Fettsäureestern, Antioxidantien, UV-Absorbern und Lichtschutzmitteln, optischen Aufhellern, Schlagzähmodifikatoren und Verarbeitungshilfen, Geliermitteln, Antistatika, Biociden, Metalldesaktivatoren, Flammschutz- und Treibmitteln, Antifogagents, Kompatibilisatoren sowie Antiplateoutagents. (Vgl. "Handbook of PVC-Formulating" von E. J. Wickson, John Wiley & Sons, New York 1993). Es folgen Beispiele für derartige Zusatzstoffe:
**I. Füllstoffe:** Füllstoffe (HANDBOOK OF PVC FORMULATING E. J. Wickson,
   John Wiley & Sons, Inc., 1993, SS. 393- 449) und Verstärkungsmittel (TASCHENBUCH der KUNSTSTOFFADDITIVE, R. Gächter & H. Müller, Carl Hanser, 1990,
   SS. 549 - 615) (wie beispielsweise Calciumcarbonat, Dolomit, Wollastonit, Magnesiumoxid, Magnesiumhydroxid, Silikate, China-Clay, Talk, Glasfasern, Glaskugeln, Holzmehl, Glimmer, Metalloxide, oder Metallhydroxide, Ruß, Graphit, Gesteinsmehl, Schwerspat, Glasfasern, Talk, Kaolin und Kreide verwandt. Bevorzugt ist Kreide. Die Füllstoffe können in einer Menge von vorzugsweise mindestens
   1 Teil, beispielsweise 5 bis 200, zweckmäßig 10 bis 150 und insbesondere 15 bis
   100 Gew.-Teilen, bezogen auf 100 Gew.-Teile PVC, eingesetzt werden.
**II. Metallseifen:** Metallseifen sind in der Hauptsache Metallcarboxylate bevorzugt längerkettiger Carbonsäuren. Geläufige Beispiele sind Stearate und Laurate, auch Oleate und Salze kürzerkettiger Alkancarbonsäuren. Als Metallseifen sollen auch Alkylbenzoesäuren gelten. Als Metalle seien genannt: Li, Na, K, Mg, Ca, Sr, Ba, Zn, Al, La, Ce und Seltenerdenmetalle. Oft verwendet man sog. synergistische Mischungen wie Barium/Zink-, Magnesium/Zink-, Calcium/Zink- oder Calcium/Magnesium/Zink-Stabilisatoren. Die Metallseifen können einzeln oder in Mischungen eingesetzt werden. Eine Übersicht über gebräuchliche Metallseifen findet sich in Ullmanns Encyclopedia of Industrial Chemistry, 5^{th} Ed., Vol. A16 (1985), S. 361 ff.). Zweckmäßig verwendet man organische Metallseifen aus der Reihe der aliphatischen gesättigten C₂-C₂₂-Carboxylate, der aliphatischen ungesättigten
   C₃-C₂₂-Carboxylate, der aliphatischen C₂-C₂₂-Carboxylate, die mit wenigstens einer OH-Gruppe substituiert sind, der cyclischen und bicyclischen Carboxylate mit
   5 - 22 C-Atomen, der unsubstituierten, mit wenigstens einer OH-Gruppe substituierten und/oder C₁-C₁₆-alkylsubstituierten Benzolcarboxylate, der unsubstituierten, mit wenigstens einer OH-Gruppe substituierten und/oder C₁-C₁₆-alkylsubstituierten Naphthalincarboxylate, der
   Phenyl-C₁-C₁₆-alkylcarboxylate, der Naphthyl-C₁-C₁₆-alkylcarboxylate oder der gegebenenfalls mit C₁-C₁₂-Alkyl substituierten Phenolate, Tallate und Resinate.
   Namentlich zu erwähnen sind, als Beispiele, die Zink-, Calcium-, Magnesiumoder Bariumsalze der monovalenten Carbonsäuren, wie Essigsäure, Propionsäure, Buttersäure, Valeriansäure, Hexansäure, Önanthsäure, Octansäure, Neodecansäure, 2-Ethylhexansäure, Pelargonsäure, Decansäure, Undecansäure, Dodecansäure, Tridecansäure, Myristylsäure, Palmitinsäure, Isostearinsäure, Stearinsäure, 12-Hydroxystearinsäure, Behensäure, Benzoesäure,
   p-tert-Butylbenzoesäure, N,N,-Dimethylhydroxybenzoesäure, 3,5-Di-tert-butyl-4-hydroxybenzoesäure, Tolylsäure, Dimethylbenzoesäure, Ethylbenzoesäure, n-Propylbenzoesäure, Salicylsäure, p-tert-Octylsalicylsäure, und Sorbinsäure; Calcium-, Magnesium- und Zinksalze der Monoester der divalenten Carbonsäuren, wie Oxalsäure, Malonsäure, Bernsteinsäure, Glutarsäure, Adipinsäure, Fumarsäure, Pentan-1,5-dicarbonsäure,
   Hexan-1,6-dicarbonsäure, Heptan-1,7-dicarbonsäure, Octan-1,8-dicarbonsäure, Phthalsäure, Isophthalsäure, Terephthalsäure und Hydroxyphthalsäure; und der Di- oder Triester der tri-oder tetravalenten Carbonsäuren, wie Hemimellithsäure, Trimellithsäure, Pyromellithsäure, Zitronensäure.
   Bevorzugt sind Calcium-, Magnesium- und Zink-Carboxylate von Carbonsäuren mit 7 bis 18 C-Atomen (Metallseifen im engeren Sinn), wie beispielsweise Benzoate oder Alkanoate, bevorzugt Stearat, Oleat, Laurat, Palmitat, Behenat, Hydroxystearate, Dihydroxystearate oder 2-Ethylhexanoat. Besonders bevorzugt sind Stearat, Oleat und p-tert-Butylbenzoat. Auch überbasische Carboxylate wie überbasisches Zinkoctoat sind bevorzugt. Ebenfalls bevorzugt sind überbasische Calciumseifen.
   Gegebenenfalls kann auch ein Gemisch von Carboxylaten unterschiedlicher Struktur eingesetzt werden.
   Bevorzugt sind Zusammensetzungen, wie beschrieben, enthaltend eine organische Zink- oder/und Calciumverbindung.
   Neben den genannten Verbindungen kommen auch organische Aluminium-Verbindungen in Frage, außerdem Verbindungen analog den oben erwähnten,
   insbesondere Aluminium-tri-stearat, Aluminium-di-stearat und Aluminium-monostearat, sowie Aluminiumacetat als auch davon abgeleitete basische Derivate. Zu den verwendbaren und bevorzugten Aluminium-Verbindungen finden sich weitere Erläuterungen in US 4,060,512 und US 3,243,394.
   Neben den bereits genannten Verbindungen kommen ferner auch organische Seltenerd-Verbindungen, insbesondere Verbindungen analog den oben erwähnten, in Frage. Unter dem Begriff Seltenerd-Verbindung sind vor allem Verbindungen der Elemente Cer, Praseodym, Neodym, Samarium, Europium, Gadolinium, Terbium, Dysprosium, Holmium, Erbium, Thulium, Ytterbium, Lutetium, Lanthan und Yttrium zu verstehen, wobei Gemische insbesondere mit Cer bevorzugt sind. Weitere bevorzugte Seltenerd-Verbindungen finden sich in der EP-A-0 108 023.
   Gegebenenfalls kann ein Gemisch von Zink-, Alkali-, Erdalkali-, Aluminium-, Cer-, Lanthan- oder Lanthanoid-Verbindung unterschiedlicher Struktur eingesetzt werden. Auch können organische Zink-, Aluminium-, Cer-, Alkali-, Erdalkali-, Lanthan- oder Lanthanoid-Verbindungen auf eine Alumosalz-Verbindung gecoatet sein; siehe hierzu auch DE-A-4 031 818.
   Die Metallseifen bzw. deren Mischungen können in einer Menge von beispielsweise 0,001 bis 10 Gew.-Teilen, zweckmäßig 0,01 bis 8 Gew.-Teilen, besonders bevorzugt 0,05 bis 5 Gew.-Teilen, bezogen auf 100 Gew.-Teile PVC, angewandt werden. Gleiches gilt für die weiteren Metallstabilisatoren:
**III. Weitere Metallstabilisatoren:** Hier sind vor allem die Organozinnstabilisatoren zu nennen. Insbesondere kann es sich um Carboxylate, Mercaptide und Sulfide handeln. Beispiele geeigneter Verbindungen sind in US 4,743,640 beschrieben.
**IV. Alkali und Erdalkali-Verbindungen:** Darunter versteht man vornehmlich die Carboxylate der oben beschriebenen Säuren, aber auch entsprechende Oxide bzw. Hydroxide oder Carbonate. Es kommen auch deren Gemische mit organischen Säuren in Frage. Beispiele sind LiOH, NaOH, KOH, CaO, Ca(OH₂), MgO, Mg(OH)₂, Sr(OH)₂, Al(OH)₃, CaCO₃ und MgCO₃ (auch basische Carbonate, wie beispielsweise Magnesia Alba und Huntit), sowie fettsaure Naund K-Salze. Bei Erdalkali- und Zn-Carboxylaten können auch deren Addukte mit MO oder M(OH)₂
   (M = Ca, Mg, Sr oder Zn), sogenannte "overbased" Verbindungen, zum Einsatz kommen. Bevorzugt werden zusätzlich zur erfindungsgemäßen Stabilisatorkombination Alkali-, Erdalkali- und/oder Aluminiumcarboxylate eingesetzt.
**V. Gleitmittel:** Als Gleitmittel kommen beispielsweise in Betracht:
   Montanwachs, Fettsäureester, PE-Wachse, Amidwachse, Chlorparaffine, Glycerinester oder Erdalkaliseifen. Verwendbare Gleitmittel sind auch in "Kunststoffadditive",
   R. Gächter/H. Müller, Carl Hanser Verlag, 3. Aufl., 1989, Seiten 478 - 488 beschrieben. Zu erwähnen sind ferner Fettketone (wie in DE 4 204 887 beschrieben) sowie Gleitmittel auf Silikonbasis (wie in EP 0 225 261 beschrieben) oder Kombinationen davon, wie in EP 0 259 783 aufgeführt. Bevorzugt ist Calciumstearat. Die Gleitmittel können auch auf eine Alumosalz-Verbindung aufgebracht werden; siehe hierzu auch DE-A-4 031 818.
**VI. Weichmacher:** Als organische Weichmacher kommen beispielsweise solche aus den folgenden Gruppen in Betracht:
   A) Phthalsäureester: Beispiele für solche Weichmacher sind Dimethyl-, Diethyl-, Dibutyl-, Dihexyl-, Di-2-ethylhexyl-, Di-n-octyl-, Di-iso-octyl-, Di-iso-nonyl-,
      Di-iso-decyl-, Di-iso-tridecyl-, Dicyclohexyl-, Di-methylcyclohexyl-, Dimethylglycol-, Dibutylglycol-, Benzylbutyl- und Diphenyl-phthalat sowie Mischungen von Phthalaten wie C₇-C₉- und C₉-C₁₁-Alkylphthalate aus überwiegend linearen Alkoholen,
      C₆-C₁₀-n-Alkylphthalate und C₈-C₁₀-n-Alkylphthalate. Bevorzugt sind davon Dibutyl-, Dihexyl-, Di-2-ethylhexyl-, Di-n-octyl-, Di-iso-octyl-, Di-iso-nonyl-, Di-iso-decyl-, Di-iso-tridecyl- und Benzylbutyl-phthalat sowie die genannten Mischungen von Alkylphthalaten. Besonders bevorzugt sind Di-2-ethylhexyl-, Di-iso-nonyl- und Di-iso-decylphthalat, die auch unter den gebräuchlichen Abkürzungen DOP (Dioctylphthalat, Di-2-ethylhexyl-phthalat), DINP (Diisononylphthalat), DIDP (Diisodecylphthalat) bekannt sind.
   B) Ester aliphatischer Dicarbonsäuren, insbesondere Ester von Adipin-, Azelain- und Sebazinsäure: Beispiele für solche Weichmacher sind Di-2-ethylhexyladipat,
      Di-isooctyladipat (Gemisch), Di-iso-nonyladipat (Gemisch), Di-iso-decyladipat (Gemisch), Benzylbutyladipat, Benzyloctyladipat, Di-2-ethylhexylazelat, Di-2-ethylhexylsebacat und Di-iso-decylsebacat (Gemisch). Bevorzugt sind Di-2-ethylhexyladipat und Di-iso-octyladipat.
   C) Trimellithsäureester, beispielsweise Tri-2-ethylhexyltrimellithat, Tri-isodecyltrimellithat (Gemisch), Tri-iso-tridecyltrimellithat, Tri-iso-octyltrimellithat (Gemisch) sowie Tri-C₆-C₈-alkyl, Tri-C₆-C₁₀-alkyl-, Tri-C₇-C₉-alkyl- und Tri-C₉-C₁₁-alkyl-trimellithate. Die letztgenannten Trimellithate entstehen durch Veresterung der Trimellithsäure mit den entsprechenden Alkanolgemischen. Bevorzugte Trimellithate sind Tri-2-ethylhexyltrimellithat und die genannten Trimellithate aus Alkanolgemischen. Gebräuchliche Abkürzungen sind TOTM (Trioctyltrimellitat, Tri-2-ethylhexyl―trimellitat), TIDTM (Triisodecyltrimellitat) und TITDTM (Triisotridecyltrimellitat).
   D) Epoxyweichmacher: In der Hauptsache sind das epoxidierte ungesättigte Fettsäuren wie z. B. epoxidiertes Sojabohnenöl.
   E) Polymerweichmacher: Eine Definition dieser Weichmacher und Beispiele für solche sind in "Kunststoffadditive", R. Gächter/H. Müller, Carl Hanser Verlag, 3. Aufl., 1989, Kapitel 5.9.6, Seiten 412 - 415, sowie in "PVC Technology", W. V. Titow,
      4^{th}. Ed., Elsevier Publ., 1984, Seiten 165 - 170 angegeben. Die gebräuchlichsten Ausgangsmaterialien für die Herstellung der Polyesterweichmacher sind:
      Dicarbonsäuren wie Adipin-, Phthal-, Azelain- und Sebacinsäure; Diole wie 1,2-Propandiol, 1,3-Butandiol, 1,4-Butandiol, 1,6-Hexandiol, Neopentylglycol und
      Diethylenglykol.
   F) Phosphorsäureester: Eine Definition dieser Ester ist im vorstehend genannten "Taschenbuch der Kunststoffadditive" Kapitel 5.9.5, SS. 408- 412, zu finden. Beispiele für solche Phosphorsäureester sind Tributylphosphat, Tri-2-ethylbutylphosphat, Tri-2-ethylhexylphosphat, Trichlorethylphosphat, 2-Ethyl-hexyl-di-phenylphosphat, Kresyldiphenylphosphat, Triphenylphosphat, Trikresylphosphat und Trixylenylphosphat. Bevorzugt sind Tri-2-ethylhexylphosphat sowie ®Reofos 50 und 95 (Ciba Spezialitätenchemie).
   G) Chlorierte Kohlenwasserstoffe (Paraffine)
   H) Kohlenwasserstoffe
   I) Monoester, z. B. Butyloleat, Phenoxyethyloleat, Tetrahydrofurfuryloleat und Alkylsulfonsäureester.
   J) Glykolester, z. B. Diglykolbenzoate.
   Definitionen und Beispiele für Weichmacher der Gruppen G) bis J) sind den folgenden Handbüchern zu entnehmen:
   "Kunststoffadditive", R. Gächter/H. Müller, Carl Hanser Verlag, 3. Aufl., 1989, Kapitel 5.9.14.2, SS.422 - 425, (Gruppe G), und Kapitel 5.9.14.1, S. 422, (Gruppe H).
   "PVC Technology", W. V. Titow, 4^{th.} Ed., Elsevier Publishers, 1984, Kapitel 6.10.2,
   Seiten 171 - 173, (Gruppe G), Kapitel 6.10.5 Seite 174, (Gruppe H), Kapitel 6.10.3,

   Seite 173, (Gruppe I) und Kapitel 6.10.4, Seiten 173 - 174 (Gruppe J). Es können auch Mischungen unterschiedlicher Weichmacher verwandt werden. Die Weichmacher können in einer Menge von beispielsweise 5 bis 20 Gew.-Teilen, zweckmäßig 10 bis 20 Gew.-Teilen, bezogen auf 100 Gew.-Teile PVC, angewandt werden. Hart- bzw. Halbhart-PVC enthält bevorzugt bis zu 10 %, besonders bevorzugt bis zu 5 % oder keinen Weichmacher.
**VII. Pigmente:** Geeignete Stoffe sind dem Fachmann bekannt. Beispiele für anorganische Pigmente sind TiO₂, Pigmente auf Zirkonoxidbasis, BaSO₄, Zinkoxid (Zinkweiss) und Lithopone (Zinksulfid/Bariumsulfat), Ruß, Russ-Titandioxid-Mischungen, Eisenoxidpigmente, Sb₂O₃, (Ti,Ba,Sb)O_{2 ,} Cr₂O₃, Spinelle wie Cobaltblau und Cobaltgrün, Cd(S,Se), Ultramarinblau. Organische Pigmente sind
   z. B. Azopigmente, Phthalocyaninpigmente, Chinacridonpigmente, Perylenpigmente, Diketo-pyrrolopyrrolpigmente und Anthrachinonpigmente. Bevorzugt ist TiO₂ auch in mikronisierter Form. Eine Definition und weitere Beschreibungen finden sich im "Handbook of PVC Formulating", E. J.Wickson, John Wiley & Sons, New York, 1993.
**VIII. Phosphite (Phosphorigsäuretriester):** Beispiele sind Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert-butylphenyl)-phosphit, Diisodecylpentaerythrit―diphosphit, Bis-(2,4-di-tertbutylphenyl)-pentaerythritdiphosphit, Bis-(2,6-di-tert―butyl-4-methylphenyl)-pentaerythritdiphosphit, Bis-isodecyloxy-pentaerythritdiphosphit, Bis-(2,4-di-tert-butyl-6-methylphenyl)-pentaerythritdiphosphit, Bis-(2,4,6-tri-tert-butylphenyl)-pentaerythritdiphosphit, Tristearyl-sorbit―triphosphit, Bis-(2,4-di-tert-butyl-6-methylphenyl)-methylphosphit, Bis-(2,4-di-tert―butyl-6-methylphenyl)-ethylphosphit. Besonders geeignete sind Trioctyl-, Tridecyl-, Tridodecyl-, Tritetradecyl, Tristearyl-, Trioleyl-, Triphenyl-, Trikresyl-, Tris-p-nonylphenyl- oder Tricyclohexylphosphit und besonders bevorzugt sind die Aryl-Dialkyl- sowie die Alkyl-Diaryl-Phosphite, wie z. B. Phenyldidecyl-, (2,4-Di-tert-butylphenyl)-didodecylphosphit, (2,6-Di-tert- butylphenyl)-di-dodecylphosphit und die Dialkylund Diaryl-pentaerythrit-diphosphite, wie Distearylpentaerythrit-diphosphit, sowie nichtstöchiometrische Triarylphosphite. z. B. der Zusammensetzung (H₁₉C₉-C₆H₄)O_{1,5}P(OC_{12,13}H_{25,27})_{1,5 oder} (H₈C₁₇-C₆H₄)O₂P(i-C₈H₁₇O) oder (H₁₉C₉-C₆H₄)O_{1,5}P(OC_{9,11}H_{19,23})_{1,5 oder} 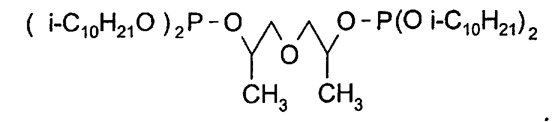 Bevorzugte organische Phosphite sind Distearyl-pentaerythrit-diphosphit, Trisnonylphenylphosphit und Phenyl-didecyl-phosphit. Als weiteres kommen Phosphorigsäurediester (mit o.g. Resten) sowie Phosphorigsäuremonoester (mit o.g. Resten) evtl. auch als Alkali-, Erdalkali-, Zink- oder Aluminiumsalz in Betracht. Diese Phosphorigsäureester können auch auf eine Alumosalz-Verbindung aufgebracht sein; siehe hierzu auch DE-A-4 031 818.
   Die organischen Phosphite können in einer Menge von beispielsweise 0,01 bis 10, zweckmäßig 0,05 bis 5 und insbesondere 0,1 bis 3 Gew.-Teilen, bezogen auf
   100 Gew.-Teile PVC, angewandt werden.
**IX. Thiophosphite und Thiophosphate:** Unter Thiophosphiten bzw.Thiophosphaten sind Verbindungen vom allgemeinen Typ: (RS)₃P, (RS)₃P=O bzw. (RS)₃P=S zu verstehen, wie sie etwa in den Patentschriften DE 2 809 492,
   EP 0 090 770 und EP 0 573 394 beschrieben werden. Beispiele für diese Verbindungen sind: Trithiohexylphosphit, Trithiooctylphosphit, Trithiolaurylphosphit, Trithiobenzylphosphit, Trithiophosphorigesäure-tris-(carbo-ioctyloxy)-methyl-ester. Trithiophosphorigsäure-tris-(carbotrimethylcyclohexyloxy)-methylester, Trithio-phosphorsäure-S,S,S-tris-(carbo-ioctyloxy)-methylester, Trithiophosphorsäure-S,S,S-tris-(carbo-2-ethylhexyloxy)-methylester, Trithiophosphorsäure-S,S,S-tris-1-(carbo-hexyloxy)-ethylester, Trithiophosphorsäure-S,S,S-tris-1-(carbo-2-ethylhexyloxy)-ethalester, Trithiophosphorsäure-S,S,S-tris-2-(carbo-2-ethylhexyloxy)-ethylester.
**X. Mercaptocarbonsäure-Ester:** Beispiele für diese Verbindungen sind:
   Ester der Thioglycolsäure, Thioäpfelsäure, Mercaptopropionsäure, der Mercaptobenzoesäuren bzw. der Thiomilchsäure, Mercaptoethylstearat und - oleat, wie sie in den Patenten FR 2 459 816, EP0 090 748, FR 2 552 440, EP 0 365 483 beschrieben sind. Die gen. Mercaptocarbonsäure-Ester umfassen auch Polyolester bzw. deren Partialester, sowie davon abgeleitete Thioether.
**XI. Epoxidierte Fettsäureester und andere Epoxidverbindungen:** Die erfindungsgemäße Stablisatorkombination kann zusätzlich vorzugsweise mindestens einen epoxidierten Fettsäureester enthalten. Es kommen dafür vor allem Ester von Fettsäuren aus natürlichen Quellen (Fettsäureglyceride), wie Sojaöl oder Rapsöl, in Frage. Es können aber auch synthetische Produkte zum Einsatz kommen, wie epoxidiertes Butyloleat. Ebenso verwendet werden können epoxidiertes Polybutadien und Polyisopren, gegebenenfalls auch in partiell hydroxylierter Form, oder Glycidylacrylat und Glycidylmethacrylat als Homo- bzw. Copolymer. Diese Epoxyverbindungen können auch auf eine Alumosalz-Verbindung aufgebracht sein; siehe hierzu auch DE-A-4 031 818.
**XII. Antioxidantien:** Als solche kommen beispielsweise in Betracht:
   Alkylierte Monophenole, z. B. 2,6-Di-tert-butyl-4-methylphenol, 2-tert-butyl-4,6-dimethylphenol, 2,6-Di-tert-butyl-4-ethylphenol, 2,6-Di-tert-butyl-4-nbutylphenol, 2,6-Di-tert―butyl-4-iso-butylphenol, 2,6-Di-cyclopentyl-4-methylphenol, 2-(alpha-Methylcyclohexyl)-4,6-dimethylphenol, 2,6-Di-octadecyl-4-methylphenol, 2,4,6-Tri-cyclohexylphenol, 2,6-Di―tert-butyl-4-methoxymethylphenol, 2,6-Di-nonyl-4-methylphenol, 2,4-Dimethyl-6-(1'-methylundec-1'-yl)-phenol, 2,4-Dimethyl-6-(1'-methyl-heptadec-1'-yl)-phenol, 2,4-Dimethyl-6-(1'-methyl-tridec-1'-yl)-phenol, Octylphenol, Nonylphenol,Dodecylphenol und Mischungen davon.
   Alkylthiomethylphenole, z. B. 2,4-Di-octylthiomethyl-6-tert-butylphenol, 2,4-Di―octylthiomethyl-6-methylphenol, 2,4-Di-octylthiomethyl-6-ethylphenol, 2,6-Didodecylthiomethyl-4-nonylphenol.
   Alkylierte Hydrochinone, z. B. 2,6-Di-tert-butyl-4-methoxyphenol, 2,5-Ditert-butyl-hydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5-Di―tert-butyl-4-hydroxyanisol, 3,5-Di-tert-butyl-4-hydroxyphenyl-stearat, Bis-(3,5-di-tert-butyl-4-hydroxyphenyl)adipat.
   Hydroxylierte Thiodiphenylether, z. B. 2,2'-Thio-bis-(6-tert-butyl-4-methylphenol), 2,2'―Thio-bis-(4-octylphenol), 4,4'-Thio-bis-(6-tert-butyl-3-methylphenol), 4,4'―Thio-bis-(6-tert-butyl-2-methylphenol), 4,4'-Thio-bis-(3,6-disec.-amylphenol), 4,4'-Bis-(2,6-dimethyl-4-hydroxyphenyl)-disulfid.
   Alkyliden-Bisphenole, z. B. 2,2'-Methylen-bis-(6-tert-butyl-4-methylphenol), 2,2'―Methylen―bis-(6-tert-butyl-4-ethylphenol), 2,2'-Methylen-bis-[4-methyl-6-(alpha―methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert-butylphenol), 2,2'-Ethyliden-bis-(4,6-di-tert-butylphenol), 2,2'-Ethyliden-bis-(6-tert-butyl-4-isobutylphenol), 2,2'-Methylen-bis-[6-(alpha-methylbenzyl)-4-nonylphenol], 2,2'-Methylen-bis-[6-(alpha,alpha-dimethylbenzyl)-4-nonylphenol], 4,4'-Methylen-bis-(2,6-di-tert-butylphenol), 4,4'-Methylen-bis-(6-tert-butyl-2-methylphenol), 1,1-Bis-(5-tert-butyl-4-hydroxy-2-methylphenyl)-butan, 2,6-Bis-(3-tert-butyl-5-methyl-2-hydroxybenzyl)-4-methylphenol, 1,1,3-Tris-(5-tert-butyl-4-hydroxy-2-methylphenyl)-butan, 1,1-Bis-(5-tert-butyl-4-hydroxy-2-methyl-phenyl)-3-n-dodecylmercaptobutan, Ethylenglycol―bis-[3,3-bis-(3'-tert-butyl-4'-hydroxyphenyl)-butyrat], Bis-(3-tert-butyl-4-hydroxy-5-methyl-phenyl)-dicyclopentadien, Bis-[2-(3'-tert-butyl-2'―hydroxy-5'-methyl-benzyl)-6-tert-butyl-4-methyl-phenyl]-terephthalat, 1,1-Bis-(3,5-dimethyl-2-hydroxyphenyl)-butan, 2,2-Bis-(3,5-di-tertbutyl-4-hydroxyphenyl)-propan, 2,2-Bis-(4-hydroxyphenyl)-propan, 2,2-Bis-(5-tert-butyl-4-hydroxy-2-methylphenyl)-4-n-dodecylmercapto-butan, 1,1,5,5-Tetra-(5-tert-butyl-4-hydroxy-2-methylphenyl)-pentan.
   Benzylverbindungen, z. B. 3,5,3',5'-Tetra-tert-butyl-4,4'-dihydroxydibenzylether, Octadecyl-4-hydroxy-3,5-dimethylbenzyl-mercaptoacetat, Tris-(3,5-di-tert-butyl-4-hydroxybenzyl)-amin, Bis-(4-tert-butyl-3-hydroxy-2,6-dimethylbenzyl)-dithioterephthalat, Bis-(3,5-di-tert-butyl-4-hydroxybenzyl)-sulfid, Isooctyl-3,5-ditert―butyl-4-hydroxybenzyl-mercaptoacetat.
   Hydroxybenzylierte Malonate, z. B. Dioctadecyl-2,2-bis-(3,5-di-tert-butyl-2-hydroxybenzyl)-malonat, Di-octadecyl-2-(3-tert-butyl-4-hydroxy-5-methylbenzyl)-malonat, Di―dodecylmercaptoethyl-2,2-bis-(3,5-di-tert-butyl-4-hydroxybenzyl)-malonat, Di-[4-(1,1,3,3-tetramethylbutyl)-phenyl]-2,2-bis-(3,5-di-tert-butyl-4-hydroxybenzyl)-malonat.
   Hydroxybenzyl-Aromaten, z. B. 1,3,5-Tris-(3,5-di-tert-butyl-4-hydroxybenzyl)-2,4,6-trimethylbenzol, 1,4-Bis-(3,5-di-tert-butyl-4-hydroxybenzyl)-2,3,5,6-tetramethylbenzol, 2,4,6-Tris-(3,5-di-tert-butyl-4-hydroxybenzyl)-phenol.
   Triazinverbindungen, z. B. 2,4-Bis-octylmercapto-6-(3,5-di-tert-butyl-4-hydroxyanilino)-1,3,5-triazin, 2-Octylmercapto-4,6-bis-(3,5-di-tert-butyl-4-hydroxyanilino)-1,3,5-triazin, 2-Octylmercapto-4,6-bis-(3,5-di-tert-butyl-4-hydroxyphenoxy)- 1,3,5-triazin, 2,4,6-Tris-(3,5-di-tert-butyl-4-hydroxyphenoxy)-1,2,3-triazin, 1,3,5-Tris-(3,5-di-tert-butyl-4-hydroxybenzyl)-isocyanurat, 1,3,5-Tris-(4-tert-butyl-3-hydroxy-2,6-dimethylbenzyl)-isocyanurat, 2,4,6-Tris-(3,5-di-tertbutyl-4-hydroxyphenylethyl)-1,3,5-triazin, 1,3,5-Tris-(3,5-di-tert-butyl-4-hydroxyphenylpropionyl)-hexahydro-1,3,5-triazin, 1,3,5-Tris-(3,5-dicyclohexyl-4-hydroxybenzyl)-isocyanurat.
   Phosphonate und Phosphonite, z. B. Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4-hydroxybenzylphosphonat, Dioctadecyl-5-tertbutyl-4-hydroxy-3-methylbenzylphosphonat, Ca-Salz des 3,5-Di-tert-butyl-4-hydroxybenzyl-phosphonsäure-monoethyleste rs, Tetrakis-(2,4-di-tert-butylphenyl)-4,4'-biphenylen―diphosphonit, 6-Isooctyloxy-2,4,8,10-tetra-tert-butyl--12H-dibenz[d,g]-1,3,2-dioxaphosphocin, 6-Fluor-2,4,8,10-tetra-tert-butyl-12-methyl-dibenz[d,g]-1,3,2-dioxaphosphocin.
   Acylaminophenole, z. B. 4-Hydroxy-laurinsäureanilid, 4-Hydroxystearinsäureanilid, N-(3,5-di-tert-butyl-4-hydroxyphenyl)-carbaminsäureoctylester.
   Ester der beta -(3,5-Di -tert-butyl-4-hydroxyphenyl)-propionsäure mit einoder mehrwertigen Alkoholen, wie z. B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Dipentaerythrit, Tris-(hydroxyethyl)-isocyanurat, N,N'―Bis-(hydroxyethyl)-oxalsäurediamid, 3-Thiaundecanol, 3-Thiapentadecanol, Trimethylhexandiol, Trimethylolpropan, Di-trimethylolpropan, 4-Hydroxymethyl-1-phospha-2,6,7-trioxabicyclo-[2.2.2]-octan.
   Ester der beta-(5-tert-Butyl-4-hydroxy-3-methylphenyl)-propionsäure mit ein- oder mehrwertigen Alkoholen, wie z. B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thia-undecanol, 3-Thiapentadecanol, Trimethylhexandiol, Trimethylolpropan, 4-Hydroxymethyl-1-phospha-2,6,7-trioxabicyclo-[2.2.2]-octan.
   Ester der beta-(3,5-Dicyclohexyl-4-hydroxyphenyl)-propionsäure mit einoder mehrwertigen Alkoholen, wie z. B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thia-undecanol, 3-Thiapentadecanol, Trimethylhexandiol, Trimethylolpropan, 4-Hydroxymethyl-1-phospha-2,6,7-trioxabicyclo-[2.2.2]-octan.
   Ester der 3,5-Di -tert-butyl-4-hydroxyphenylessigsäure mit ein- oder mehrwertigen Alkoholen, wie z. B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   Amide der beta-(3,5-Di -tert-butyl-4-hydroxyphenyl)-propionsäure, wie z. B. N,N'-Bis-(3,5-di-tert-butyl-4-hydroxyphenylpropionyl)-hexamethylendiamin, N,N'-Bis-(3,5-di-tert-butyl-4-hydroxyphenylpropionyl)-trimethylendiamin, N,N'-Bis-(3,5-di-tert-butyl-4-hydroxyphenylpropionyl)-hydrazin.
   Vitamin E (Tocopherol) und Abkömmlinge
   Bevorzugt sind Antioxidantien der Gruppen 1-5, 10 und 12 insbesondere 2,2-Bis-(4-hydroxyphenyl)-propan, Ester der 3,5-Di-tert-butyl-4-hydroxyphenylpropionsäure mit Octanol, Octadecanol oder Pentaerythrit oder Tris-(2,4-di-tert-butylphenyl)-phosphit.
   Gegebenenfalls kann auch ein Gemisch von Antioxidantien unterschiedlicher Struktur eingesetzt werden.
   Die Antioxidantien können in einer Menge von beispielsweise 0,01 bis 10 Gew.-Teilen, zweckmäßig 0,1 bis 10 Gew.-Teilen und insbesondere 0,1 bis 5 Gew.-Teilen, bezogen auf 100 Gew.-Teile PVC, angewandt werden.
**XIII. UV-Absorber und Lichtschutzmittel:** Beispiele dafür sind:
   2-(2'-Hydroxyphenyl)-benztriazole, wie z. B. 2-(2'-Hydroxy-5'-methylphenyl)-benztriazol, 2-(3',5'-Di-tert-butyl-2'-hydroxyphenyl)-benztriazol, 2-(5'-tert-Butyl-2'hydroxyphenyl)-benztriazol, 2-(2'-Hydroxy-5'-(1,1,3,3-tetramethylbutyl)phenyl)-benztriazol, 2-(3',5'-Di-tert-butyl-2'-hydroxyphenyl)-5-chlor-benztriazol, 2-(3'-tert-Butyl- 2'-hydroxy-5'-methylphenyl)-5-Chlor-benztriazol, 2-(3'-sec-Butyl-5'-tert-butyl-2'-hydroxyphenyl)-benztriazol, 2-(2'-Hydroxy-4'-octoxyphenyl)-benztriazol, 2-(3',5'-Di-tert-amyl-2'-hydroxyphenyl)-benztriazol, 2-(3',5'-Bis-(alpha,alpha―dimethylbenzyl)-2'-hydroxyphenyl)-benztriazol, Mischung aus 2-(3'-tert-Butyl-2'hydroxy-5'-(2-octyloxycarbonylethyl)phenyl)-5-chlor-benztriazol, 2-(3'-tert-Butyl-5'-[2-(2-ethylhexyloxy)-carbonylethyl]-2'-hydroxyphenyl)-5-chlor-benztriazol, 2-(3'-tert-Butyl-2'-hydroxy-5'-(2-methoxycarbonylethyl)phenyl)-5-chlor-benztriazol, 2-(3'-tert-Butyl-2'-hydroxy-5'-(2-methoxycarbonylethyl)phenyl)-benztriazol, 2-(3'tert-Butyl-2'-hydroxy-5'-(2-octyloxycarbonylethyl)phenyl)-benztriazol, 2-(3'-tert-Butyl-5'-[2-(2-ethylhexyloxy)carbonylethyl]-2'-hydroxyphenyl)-benztriazol, 2-(3'-Dodecyl-2'-hydroxy-5'―methylphenyl)-benztriazol, und 2-(3'-tert-Butyl-2'-hydroxy-5'-(2-isooctyloxycarbonylethyl)phenyl-benztriazol, 2,2'-Methylen-bis[4-(1,1,3,3-tetramethylbutyl)-6-benztriazol-2-yl-phenol]; Umesterungsprodukt von 2-[3'-tert-Butyl-5'-(2-methoxycarbonylethyl)-2'-hydroxy―phenyl]-benztriazol mit Polyethylenglycol 300; 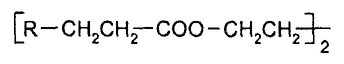 mit R = 3'-tert-Butyl-4'hydroxy-5'-2H-benztriazol-2-yl-phenyl.
   2-Hydroxybenzophenone, wie z. B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.
   Ester von gegebenenfalls substituierten Benzoesäuren, wie z. B. 4-tert--Butyl―phenylsalicylat, Phenylsalicylat, Octylphenyl-salicylat, Dibenzoylresorcin, Bis-(4-tert-butylbenzoyl)-resorcin, Benzoylresorcin, 3,5-Di-tert-butyl-4-hydroxybenzoesäure-2,4-di-tert-butylphenylester, 3,5-Di-tert-butyl-4-hydroxybenzoesäurehexadecylester, 3,5-Di-tert-butyl-4-hydroxybenzoesäureoctadecylester, 3,5-Di-tert-butyl-4-hydroxybenzoesäure-2-methyl-4,6-di-tertbutylphenylester.
   Acrylate, wie z. B. alpha-Cyan-beta,beta-diphenylacrylsäure-ethylester bzw. -isooctylester, alpha-Carbomethoxy-zimtsäuremethylester, alpha-Cyano-betamethyl-p-methoxy-zimtsäuremethylester bzw. -butylester, alpha-Carbomethoxyp-methoxy-zimtsäure-methylester, N-(beta-Carbomethoxy-b-cyanovinyl)-2-methyl-indolin.
   Nickelverbindungen, wie z. B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl―diethanolamin, Nickeldibutyldithiocarbamat, Nickelsalze von 4-Hydroxy-3,5-ditert-butylbenzylphosphonsäure―monoalkylestern, wie vom Methyl- oder Ethylester, Nickelkomplexe von Ketoximen, wie von 2-Hydroxy-4-methyl-phenylundecylketoxim, Nickelkomplexe des 1-Phenyl-4-lauroyl-5-hydroxy-pyrazols, gegebenenfalls mit zusätzlichen Liganden.
   Oxalsäurediamide, wie z. B. 4,4'-Di-octyloxy-oxanilid, 2,2'-Di-octyloxy-5,5'di-tert―butyl―oxanilid, 2,2'-Di-dodecyloxy-5,5'di-tert-butyl-oxanilid, 2-Ethoxy-2'ethyl―oxanilid, N,N'-Bis-(3-dimethylaminopropyl)-oxalamid, 2-Ethoxy-5-tertbutyl-2'-ethyloxanilid und dessen Gemisch mit 2-Ethoxy-2'-ethyl-5,4'-di-tert-butyloxanilid, Gemische von o- und p-Methoxy- sowie von o- und p-Ethoxy-disubstituierten Oxaniliden.
   2-(2-Hydroxyphenyl)-1,3,5-triazine, wie z. B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-[2-hydroxy-4-(2-hydroxy-3-butyloxy-propyloxy)phenyl]-4,6-bis(2,4-dimethylphenyl)-1,3,5-triazin, 2-[2-hydroxy-4-(2-hydroxy--3-octyloxy―propyloxy)phenyl]-4,6-bis(2,4-dimethylphenyl)-1,3,5-triazin.
   Sterisch gehinderte Amine, wie z. B. Bis(2,2,6,6-tetramethyl-piperidin-4-yl)-sebacat, Bis(2,2,6,6-tetramethyl-piperidin-4-yl)-succinat, Bis(1,2,2,6,6-pentamethylpiperidin-4-yl)-sebacat, Bis(1-octyloxy-2,2,6,6-tetramethylpiperidin-4-yl)-sebacat, n-Butyl-3,5-di-tert-butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, lineare oder cyclische Kondensationsprodukte aus N,N'-Bis(2,2,6,6-Tetramethyl-4-piperidyl)-hexamethylendiamin und 4-tert-Octylamino-2,6-dichlor-1,3,5-s-triazin, Tris(2,2,6,6-tetramethyl-4-piperidyl)-nitrilotriacetat, Tetrakis(2,2,6,6-tetramethyl-4-piperidyl)-1,2,3,4-butantetraoat, 1,1'-(1,2-Ethandiyl)-bis(3,3,5,5-tetramethylpiperazinon), 4-Benzoyl-2,2,6,6-tetramethylpiperidin, 4-Stearyloxy-2,2,6,6-tetramethylpiperidin, Bis(1,2,2,6,6-pentamethylpiperidyl)-2-n-butyl-2-(2-hydroxy-3,5-di-tert-butylbenzyl)-malonat, 3-n-Octyl-7,7,9,9-tetramethyl-1,3,8-triazaspiro[4.5]decan-2,4-dion, Bis(1-octyloxy-2,2,6,6-tetramethylpiperidyl)-sebacat, Bis(1-octyloxy-2,2,6,6-tetramethylpiperidyl)-succinat, lineare oder cyclische Kondensationsprodukte aus N,N'-Bis(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis(3-aminopropylamino)ethan, Kondensationsprodukt aus 2-Chlor-4,6-di-(4-n-butylamino-1,2,2,6,6-pentamethylpiperidyl)-1,3,5-triazin und 1,2-Bis(3-aminopropylamino)-ethan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion, Gemisch von 4-Hexadecyloxy- und 4-Stearyloxy-2,2,6,6-tetramethylpiperidin, Kondensationsprodukt aus N,N'-Bis(2,2,6,6-tetramethyl-4-piperidyl)-hexamethylendiamin und 4-Cyclohexylamino-2,6-dichlor-1,3,5-triazin, Kondensationsprodukt aus 1,2-Bis(3-aminopropylamino)-ethan und 2,4,6-trichlor-1,3,5-triazin sowie 4-Butylamino-2,2,6,6-tetramethyl-piperidin (CAS Reg. No. [136504-96-6]); N-(2,2,6,6-tetramethyl-4-piperidyl)-n-dodecylsuccinimid, N-(1,2,2,6,6-pentamethyl-4-piperidyl)-n-dodecylsuccinimid, 2-Undecyl-7,7,9,9-tetramethyl-1-oxa-3,8-diaza-4-oxo-spiro[4,5]decan, Umsetzungsprodukt von 7,7,9,9-Tetramethyl-2-cycloundecyl-1-oxa-3,8-diaza-4-oxospiro[4,5]decan und Epichlorhydrin, 1,1-Bis(1,2,2,6,6-pentamethyl-4-piperidyloxycarbonyl)-2-(4-methoxyphenyl)-ethen, N,N'-Bis-formyl-N,N'-bis(2,2,6,6-tetramethyl-4-piperidyl)-hexamethylendiamin, Diester der 4-Methoxy-methylen-malonsäure mit 1,2,2,6,6-Pentamethyl-4-hydroxy-piperidin, Poly[methylpropyl-3-oxy-4-(2,2,6,6-tetramethyl-4-piperidyl)]-siloxan, Reaktionsprodukt aus Maleinsäureanhydrid-a-olefincopolymer und 2,2,6,6-Tetramethyl-4-aminopiperidin oder 1,2,2,6,6-Pentamethyl-4-aminopiperidin.
**XIV. Treibmittel:** Treibmittel sind z. B. organische Azo- und Hydrazoverbindungen, Tetrazole, Oxazine, Isatosäureanhydrid, sowie Soda und Natriumbicarbonat. Bevorzugt sind Azodicarbonamid und Natriumbicarbonat sowie deren Mischungen.
   Definitionen und Beispiele für Schlagzähmodifikatoren und Verarbeitungshilfen, Geliermittel, Antistatika, Biocide, Metalldesaktivatoren, optische Aufheller, Flammschutzmittel, Antifogging-agents sowie Kompatibilisatoren sind beschrieben in "Kunststoffadditive", R. Gächter/H. Müller, Carl Hanser Verlag, 3. Aufl., 1989, und im "Handbook of Polyvinyl Chloride Formulating" E. J. Wickson, J. Wiley & Sons, 1993, sowie in "Plastics Additives" G. Pritchard, Chapman & Hall, London, 1st Ed., 1998.
   Schlagzähmodifikatoren sind ferner ausführlich beschrieben in "Impact Modifiers for PVC", J. T. Lutz/D. L. Dunkelberger, John Wiley & Sons, 1992.
**XV. beta-Diketone, beta-Ketoester:** Verwendbare 1,3-Dicarbonylverbindungen können lineare oder cyclische Dicarbonylverbindungen sein. Bevorzugt werden Dicarbonylverbindungen der folgenden Formel verwandt: R'₁ CO CHR₂'-COR'₃
worin R'₁ C₁-C₂₂-Alkyl, C₅-C₁₀-Hydroxyalkyl, C₂-C₁₈-Alkenyl, Phenyl, durch OH, C₁-C₄-Alkyl, C₁-C₄-Alkoxy oder Halogen substituiertes Phenyl, C₇-C₁₀-Phenylalkyl, C₅-C₁₂-Cycloalkyl, durch C₁-C₄-Alkyl substituiertes C₅-C₁₂-Cycloalkyl oder eine Gruppe -R'₅-S-R'₆ oder -R'₅-O-R'₆ bedeutet, R'₂ Wasserstoff, C₁-C₈-Alkyl,
C₂-C₁₂-Alkenyl, Phenyl, C₇-C₁₂-Alkylphenyl, C₇-C₁₀-Phenylalkyl oder eine Gruppe -CO-R'₄ bedeutet, R'₃ eine der für R'₁ gegebenen Bedeutungen hat oder
C₁-C₁₈-Alkoxy bedeutet, R'₄ C₁-C₄-Alkyl oder Phenyl bedeutet,
R'₅ C₁-C₁₀-Alkylen bedeutet und R'₆ C₁-C₁₂-Alkyl, Phenyl, C₇-C₁₈-Alkylphenyl oder C₇-C₁₀-Phenylalkyl bedeutet.
Hierzu gehören die Hydroxylgruppen enthaltenden Diketone der EP 0 346 279 und die Oxa- und Thia-diketone der EP 0 307 358 ebenso wie die auf Isocyansäure basierenden Ketoester der US 4,339,383.
R'₁ und R'₃ als Alkyl können insbesondere C₁-C₁₈-Alkyl sein, wie z. B. Methyl, Ethyl, n-Propyl, Isopropyl, n-Butyl, tert Butyl, Pentyl, Hexyl, Heptyl, Octyl, Decyl, Dodecyl oder Octadecyl.
R'₁ und R'₃ als Hydroxyalkyl stellen insbesondere eine Gruppe -(CH₂)ₙ-OH dar, worin n 5, 6 oder 7 ist.
R'₁ und R'₃ als Alkenyl können beispielsweise Vinyl, Allyl, Methallyl, 1-Butenyl, 1-Hexenyl oder Oleyl bedeuten, vorzugsweise Allyl.
R'₁ und R'₃ als durch OH, Alkyl, Alkoxy oder Halogen substituiertes Phenyl können beispielsweise Tolyl, Xylyl, tert Butylphenyl, Methoxyphenyl, Ethoxyphenyl, Hydroxyphenyl, Chlorphenyl oder Dichlorphenyl sein.
R'₁ und R'₃ als Phenylalkyl sind insbesondere Benzyl. R'₂ und R'₃ als Cycloalkyl oder Alkyl―cycloalkyl sind insbesondere Cyclohexyl oder Methylcyclohexyl.
R'₂ als Alkyl kann insbesondere C₁-C₄-Alkyl sein. R'₂ als C₂-C₁₂-Alkenyl kann insbesondere Allyl sein. R'₂ als Alkylphenyl kann insbesondere Tolyl sein. R'₂ als Phenylalkyl kann insbesondere Benzyl sein. Vorzugsweise ist R'₂ Wasserstoff. R'₃ als Alkoxy kann z. B. Methoxy, Ethoxy, Butoxy, Hexyloxy, Octyloxy, Dodecyloxy, Tridecyloxy, Tetradecyloxy oder Octadecyloxy sein. R'₅ als C₁-C₁₀-Alkylen ist insbesondere C₂-C₄-Alkylen. R'₆ als Alkyl ist insbesondere C₄-C₁₂-Alkyl, wie z. B. Butyl, Hexyl, Octyl, Decyl oder Dodecyl. R'₆ als Alkylphenyl ist insbesondere Tolyl. R'₆ als Phenylalkyl ist insbesondere Benzyl.
Beispiele für 1,3-Dicarbonylverbindungen der obigen Formel sowie deren Alkali-, Erdalkali- und Zinkchelate sind Acetylaceton, Butanoylaceton, Heptanoylaceton, Stearoylaceton, Palmitoylaceton, Lauroylaceton, 7-tert.-Nonylthio-heptandion-2,4, Benzoylaceton, Dibenzoylmethan, Lauroylbenzoylmethan, Palmitoylbenzoylmethan, Stearoyl-benzoylmethan, Isooctylbenzoylmethan, 5-Hydroxycapronyl-benzoylmethan, Tribenzoylmethan, Bis(4-methylbenzoyl)methan, Benzoyl-p-chlorbenzoylmethan, Bis(2-hydroxybenzoyl)methan, 4-Methoxybenzoyl-benzoylmethan, Bis(4-methoxybenzoyl)-methan, 1-Benzoyl-1-acetylnonan, Benzoyl-acetyl-phenylmethan, Stearoyl-4-methoxybenzoylmethan, Bis(4-tert-butylbenzoyl)methan, Benzoyl-formylmethan, Benzoyl-phenylacetylmethan, Bis-cyclohexanoyl-methan, Di-pivaloyl-methan, 2-Acetylcyclopentanon, 2-Benzoylcyclo-pentanon, Diacetessigsäuremethyl-, -ethylund -allylester, Benzoyl-, Propionyl- und Butyryl-acetessigsäuremethyl- und -ethylester, Triacetylmethan, Acetessigsäuremethyl-, -ethyl-, -hexyl-, -octyl-, -dodecyl- oder -octadecylester, Benzoylessigsäuremethyl-, -ethyl-, -butyl-, -2-ethylhexyl-, -dodecyl- oder -octadecylester, sowie Propionyl- und Butyrylessigsäure-C₁-C₁₈-alkylester. Stearoylessigsäureethyl-, -propyl-, -butyl-, -hexyl- oder -octylester sowie mehrkernige β-Ketoester wie in EP 0 433 230 beschrieben und Dehydracetsäure sowie deren Zink-, Magnesium- oder Alkalisalze.
Bevorzugt sind 1,3-Diketoverbindungen der obigen Formel, worin R'₁ C₁-C₁₈-Alkyl, Phenyl, durch OH, Methyl oder Methoxy substituiertes Phenyl, C₇-C₁₀-Phenylalkyl oder Cyclohexyl ist, R'₂ Wasserstoff ist und R'₃ eine der für R'₁ gegebenen Bedeutungen hat.

Die 1,3-Diketoverbindungen können in einer Menge von beispielsweise 0,01 bis 10, zweckmäßig 0,01 bis 3 und insbesondere 0,01 bis 2 Gew.-Teilen, bezogen auf
100 Gew.-Teile PVC, angewandt werden.

Beispiele für die zu stabilisierenden chlorhaltige Polymere sind: Polymere des Vinylchlorides, Vinylidenchlorids, Vinylharze, enthaltend Vinylchlorideinheiten in deren Struktur, wie Copolymere des Vinylchlorids und Vinylester von aliphatischen Säuren, insbesondere Vinylacetat, Copolymere des Vinylchlorids mit Estern der Acryl- und Methycrylsäure und mit Acrylnitril, Copolymere des Vinylchlorids mit Dienverbindungen und ungesättigten Dicarbonsäuren oder deren Anhydride, wie Copolymere des Vinylchlorids mit Diethylmaleat, Diethylfumarat oder Maleinsäureanhydrid, nachchlorierte Polymere und Copolymere des Vinylchlorids, Copolymere des Vinylchlorids und Vinylidenchlorids mit ungesättigten Aldehyden, Ketonen und anderen, wie Acrolein, Crotonaldehyd, Vinylmethylketon, Vinylmethylether, Vinylisobutylether und ähnliche; Polymere des Vinylidenchlorids und Copolymere desselben mit Vinylchlorid und anderen polymerisierbaren Verbindungen; Polymere des Vinylchloracetates und Dichlordivinylethers; chlorierte Polymere des Vinylacetates, chlorierte polymerische Ester der Acrylsäure und der alphasubstituierten Acrylsäure; Polymere von chlorierten Styrolen, zum Beispiel Dichlorstyrol; chlorierte Gummis; chlorierte Polymere des Ethylens; Polymere und nachchlorierte Polymere von Chlorbutadiens und deren Copolymere mit Vinylchlorid, chlorierte Natur- und Synthesekautschuke, sowie Mischungen der genannten Polymere unter sich oder mit anderen polymerisierbaren Verbindungen. Im Rahmen dieser Erfindung sind unter PVC auch Copolymerisate mit polymerisierbaren Verbindungen wie Acrylnitril, Vinylacetat oder ABS zu verstehen, wobei es sich um Suspensions-, Masse- oder Emulsionspolymerisate handeln kann. Bevorzugt ist ein PVC-Homopolymer, auch in Kombination mit Polyacrylaten.
Ferner kommen auch Pfropfpolymerisate von PVC mit EVA, ABS und MBS in Betracht. Bevorzugte Substrate sind auch Mischungen der vorstehend genannten Homo-und Copolymerisate, insbesondere Vinylchlorid-Homopolymerisate, mit anderen thermoplastischen oder/und elastomeren Polymeren, insbesondere Blends mit ABS, MBS, NBR, SAN, EVA, CPE, MBAS, PMA, PMMA, EPDM und Polylactonen.
Beispiele für solche Komponenten sind Zusammensetzungen aus (i) 20-80 Gew.-Teilen eines Vinylchlorid-Homopolymeren (PVC) und (ii) 80-20 Gew.-Teile mindestens eines thermoplastischen Copolymerisats auf der Basis von Styrol und Acrylnitril, insbesondere aus der Gruppe ABS, NBR, NAR, SAN und EVA. Die verwandten Abkürzungen für die Copolymerisate sind dem Fachmann geläufig und bedeuten folgendes: ABS: Acrylnitril-Butadien-Styrol; SAN: Styrol-Acrylnitril; NBR: Acrylnitril-Butadien; NAR: Acrylnitril-Acrylat; EVA: EthylenVinylacetat. Es kommen insbesondere auch Styrol-Acrylnitril-Copolymerisate auf Acrylat-Basis (ASA) in Betracht. Bevorzugt als Komponente sind in diesem Zusammenhang Polymerzusammensetzungen, die als Komponenten (i) und (ii) eine Mischung aus
25 - 75 Gew.-% PVC und 75 - 25 Gew.-% der genannten Copolymerisate enthalten. Beispiele für solche Zusammensetzungen sind: 25 - 50 Gew.-% PVC und
75 - 50 Gew.-% Copolymerisate bzw. 40 - 75 Gew.-% PVC und 60 - 25 Gew.-% Copolymerisate. Bevorzugte Copolymerisate sind ABS, SAN und modifiziertes EVA, insbesondere ABS. Besonders geeignet sind auch NBR, NAR und EVA. In der erfindungsgemäßen Zusammensetzung können eines oder mehrere der genannten Copolymerisate vorhanden sein. Von besonderer Bedeutung sind als Komponente Zusammensetzungen, die (i) 100 Gewichtsteile PVC, und (ii) 0 - 300 Gewichtsteile ABS und/oder mit SAN modifiziertes ABS und 0 - 80 Gewichtsteile der Copolymeren NBR, NAR und/oder EVA, insbesondere jedoch EVA, enthalten.
Weiterhin kommen zur Stabilisierung im Rahmen dieser Erfindung auch insbesondere Recyclate chlorhaltiger Polymere in Frage, wobei es sich hierbei um die oben näher beschriebenen Polymere handelt, welche durch Verarbeitung, Gebrauch oder Lagerung eine Schädigung erfahren haben. Besonders bevorzugt ist PVC-Recyclat. In den Recyclaten können auch kleine Mengen an Fremdstoffen enthalten sein, wie z. B. Papier, Pigmente, Klebstoffe, die oft schwierig zu entfernen sind. Diese Fremdstoffe können auch aus dem Kontakt mit diversen Stoffen während des Gebrauchs oder der Aufarbeitung stammen, wie z. B. Treibstoffreste, Lackanteile, Metallspuren und Initiatorreste.

Die erfindungsgemäße Stabilisierung ist besonders bei PVC-Formulierungen von Vorteil, wie sie für Rohre und Profile üblich sind. Die Stabilisierung kann ohne Schwermetallverbindungen (Sn-, Pb-, Cd-, Zn-Stabilisatoren) durchgeführt werden. Diese Eigenschaft bietet auf bestimmten Gebieten Vorteile, weil Schwermetalle - mit Ausnahmen von allenfalls Zink - sowohl bei der Produktion als auch bei der Anwendung bestimmter PVC-Artikel aus ökologischen Gründen oft unerwünscht sind. Auch bereitet die Herstellung von Schwermetallstabilisatoren aus gewerbehygienischer Hinsicht oftmals Probleme. Ebenfalls ist die Verhüttung von schwermetallhaltigen Erzen sehr oft mit gravierenden Einflüssen auf die Umwelt verbunden, wobei Umwelt das Biosystem Mensch, Tier (Fisch), Pflanze, Luft und Boden mit einschließt. Aus diesen Gründen ist auch die Verbrennung bzw. Deponierung von schwermetallhaltigen Kunststoffen umstritten.

Die Erfindung betrifft auch ein Verfahren zur Stabilisierung von PVC, dadurch gekennzeichnet, daß man diesem mindestens eine der oben erwähnten Stabilisatorkombinationen zufügt.

Zweckmäßig kann die Einarbeitung der Stabilisatoren nach folgenden Methoden erfolgen: als Emulsion oder Dispersion (Eine Möglichkeit ist z. B. die Form einer pastösen Mischung. Ein Vorteil der erfindungsgemäßen Kombination besteht bei dieser Darreichungsform in der Stabilität der Paste.); als Trockenmischung während des Vermischens von Zusatzkomponenten oder Polymermischungen; durch direktes Zugeben in die Verarbeitungsapparatur (z. B. Kalander, Mischer, Kneter, Extruder und dergleichen) oder als Lösung oder Schmelze bzw. als Flakes oder Pellets in staubfreier Form als One- Pack.

Das erfindungsgemäß stabilisierte PVC, das die Erfindung ebenfalls betrifft, kann auf an sich bekannte Weise hergestellt werden, wozu man unter Verwendung an sich bekannter Vorrichtungen wie der oben genannten Verarbeitungsapparaturen die erfindungsgemäße Stabilisatorkombination und gegebenenfalls weitere Zusätze mit dem PVC vermischt. Hierbei können die Stabilisatoren einzeln oder in Mischung zugegeben werden oder auch in Form sogenannter Masterbatches.
Das nach vorliegender Erfindung stabilisierte PVC kann auf bekannte Weisen in die gewünschte Form gebracht werden. Solche Verfahren sind beispielsweise Mahlen, Kalandrieren, Extrudieren, Spritzgießen oder Spinnen, ferner Extrusions-Blasen. Das stabilisierte PVC kann auch zu Schaumstoffen verarbeitet werden.
Ein erfindungsgemäß stabilisiertes PVC eignet sich z. B. besonders für Hohlkörper (Flaschen), Verpackungsfolien (Tiefziehfolien), Blasfolien, Rohre, Schaumstoffe, Schwerprofile (Fensterrahmen), Lichtwandprofile, Bauprofile, Sidings, Fittings, Bürofolien und Apparatur-Gehäuse (Computer, Haushaltsgeräte).
Bevorzugt sind PVC-Hartschaumstoff-Formkörper und PVC-Rohre wie für Trinkoder Abwasser, Druckrohre, Gasrohre, Kabelkanal- und Kabelschutzrohre, Rohre für Industrieleitungen, Sickerrohre, Abflußrohre, Dachrinnenrohre und Drainagerohre. Näheres hierzu siehe "Kunststoffhandbuch PVC", Band 2/2, W. Becker/H. Braun,
2. Aufl., 1985, Carl Hanser Verlag, Seiten 1236 - 1277.

6-Aminouracile werden nach bekannten Methoden hergestellt [z. B. US Patent 2,598,936, WO 96/04280, J. Org. Chem. **16,** 1879 - 1890 (1951), J. Org. Chem. **30,** 656 (1965), JACS **82,** 3973 (1960) und Synthesis 1996, Seite 459 ff.].
Dargestellte Verbindungen sind in der **Tabelle 1** zusammengefasst. Teile und Prozentangaben beziehen sich wie im übrigen Text auf das Gewicht, sofern nicht anders angegeben.

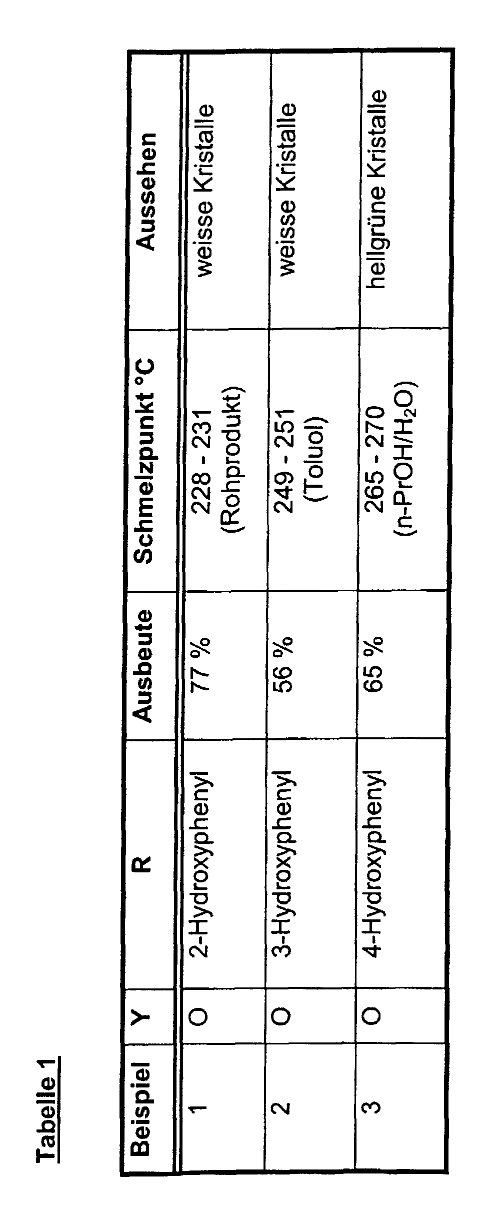

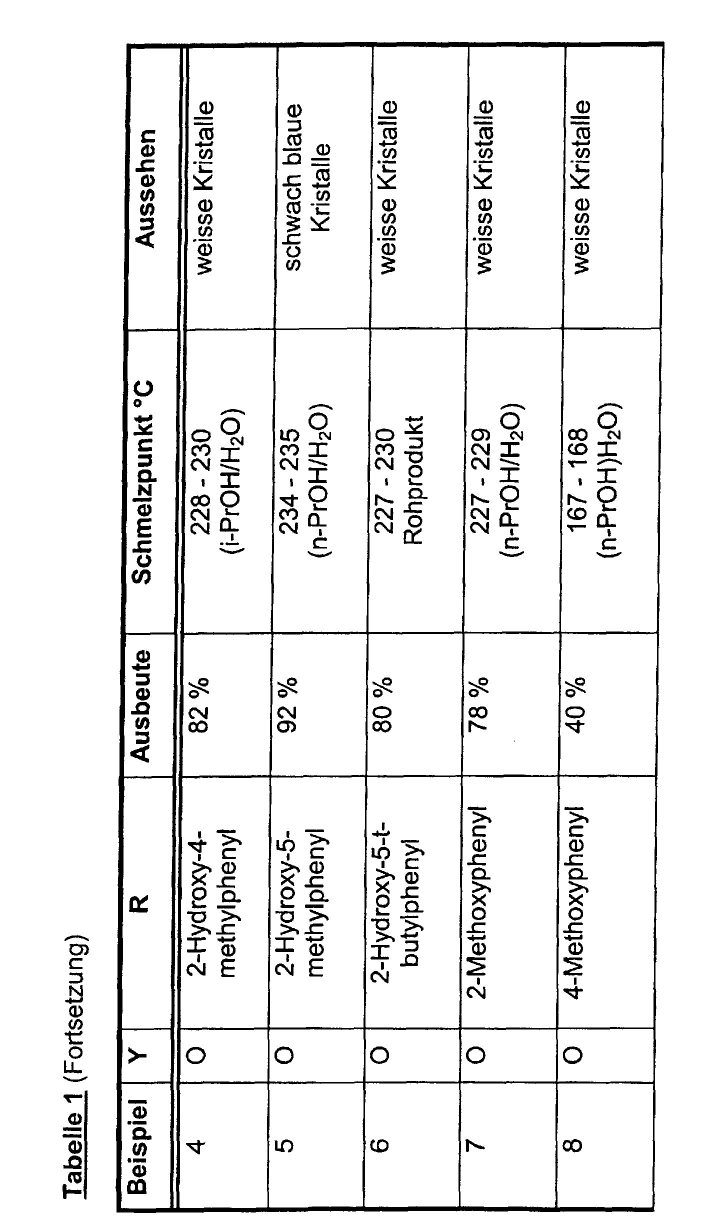

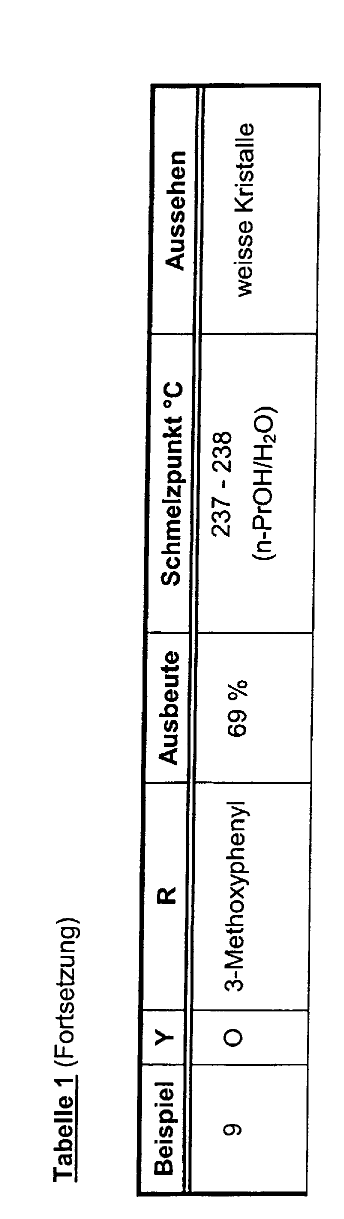

### Herstellungsbeispiele

### Beispiel 1

In einem 1 I 4-Halskolben mit KPG-Rührer, Luftkühler, Thermometer und Blasenzähler werden unter Stickstoffatmosphäre 155,2 g (1,0 Mol) 6-Amino-1,3-dimethyluracil, 160 g (1,1 Mol) 2-Aminophenol-Hydrochlorid und 300 g Diethylenglykoldimethylether vorgelegt und 75 Minuten lang bei 145 - 155 °C gerührt. Anschliessend wird das Reaktionsgemisch auf 10 °C abgekühlt, die ausgefallenen Kristalle auf einer Nutsche abgesaugt und zweimal mit je 100 ml i-Propanol gewaschen. Die Kristalle werden bei 80 °C im Vakuumtrockenschrank getrocknet. Dieser getrocknete Rückstand von 244,1 g wird dann mit 300 g Wasser 30 Minuten lang gerührt, das Reaktionsprodukt abgesaugt, mit 100 g Wasser gewaschen und getrocknet.

Ausbeute: 188,6 g (≅ 76,3 % d. Th.) weisses, kristallines Pulver (Smp. 228 - 230°C)

### Beispiel 8:

Ein Gemisch aus 43,5 g (0,357 Mol) p-Anisidin, 27,3 g (0,176 Mol) 6-Amino-1,3-dimethyluracil und 20 g 32 %-iger Salzsäure wird innerhalb 1,5 Stunden auf 150 °C unter Rühren erwärmt. Während der Aufheizphase wird das Wasser abdestilliert. Das Reaktionsgemisch wird weitere 1,5 Stunden bei 150 °C gerührt, anschliessend auf 90 °C abgekühlt, mit 100 ml Methanol versetzt, 30 Minuten auf Rückfluss erwärmt und dann auf 5 °C abgekühlt. Der kristalline Niederschlag wird abgesaugt und anschliessend aus einem Gemisch von
200 ml n-Propanol/60 ml Wasser umkristallisiert.
Ausbeute: 18,3 g (≅ 40 % d. Th.) weisse Kristalle (Smp. 167 - 168°C)

### Statischer Hitzetest

Die Mischungen gemäß der nachfolgenden Tabellen und Legenden werden jeweils 5 Minuten bei 180°C bzw. 190°C auf einem Mischwalzwerk plastifiziert. Aus den erhaltenen Folien (Stärke 0,3 mm) werden Prüfstreifen geschnitten und diese in einem Mathis-Thermo-Takter bei 190°C über den in der nachstehenden Tabelle angegebenen Zeitraum thermisch belastet. Anschliessend wird der Yellowness Index (YI) nach ASTM-1925-70 bestimmt.
Je niedriger der gefundene YI-Wert ist, desto wirksamer ist das Stabilisatorsystem.

### Beispiel I

Die Ergebnisse sind der folgenden Tabelle 2 zu entnehmen. Geringe **YI-**Werte bedeuten gute Stabilisierung.

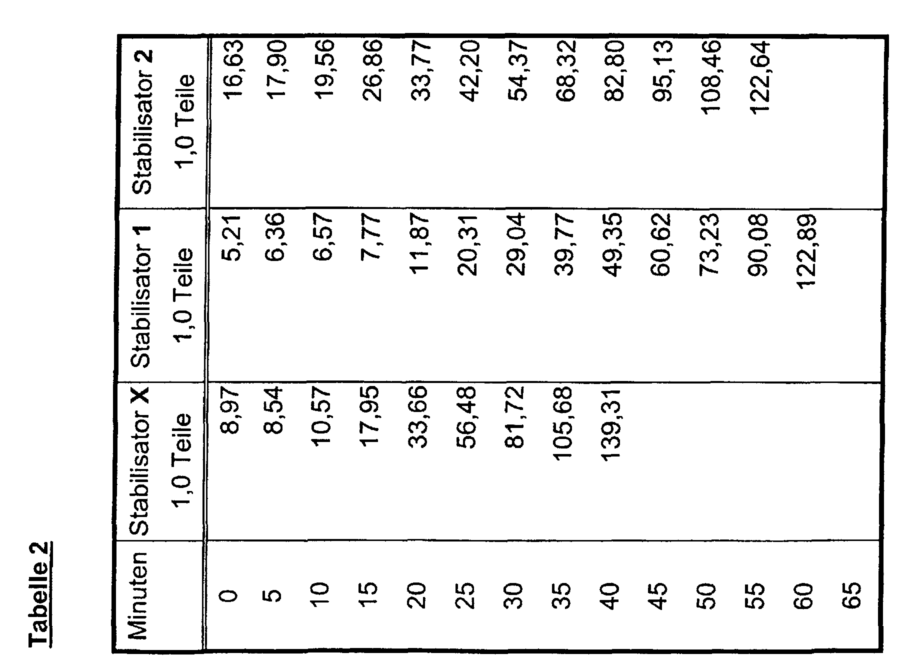

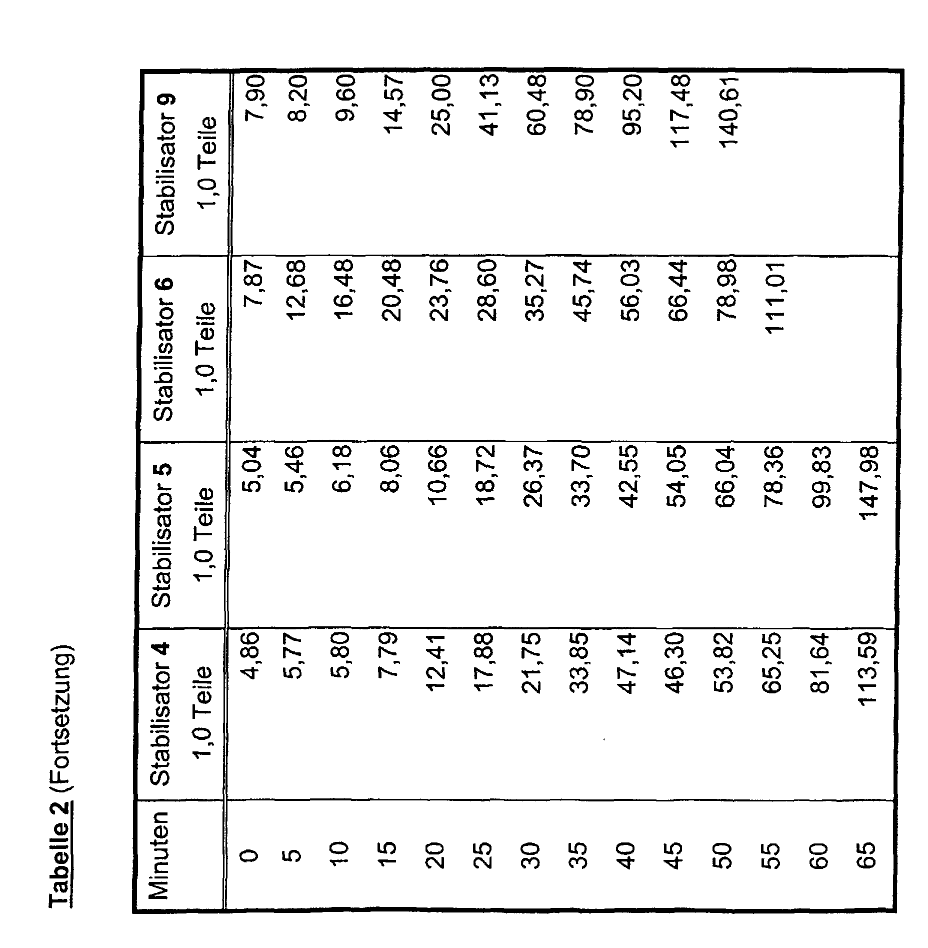

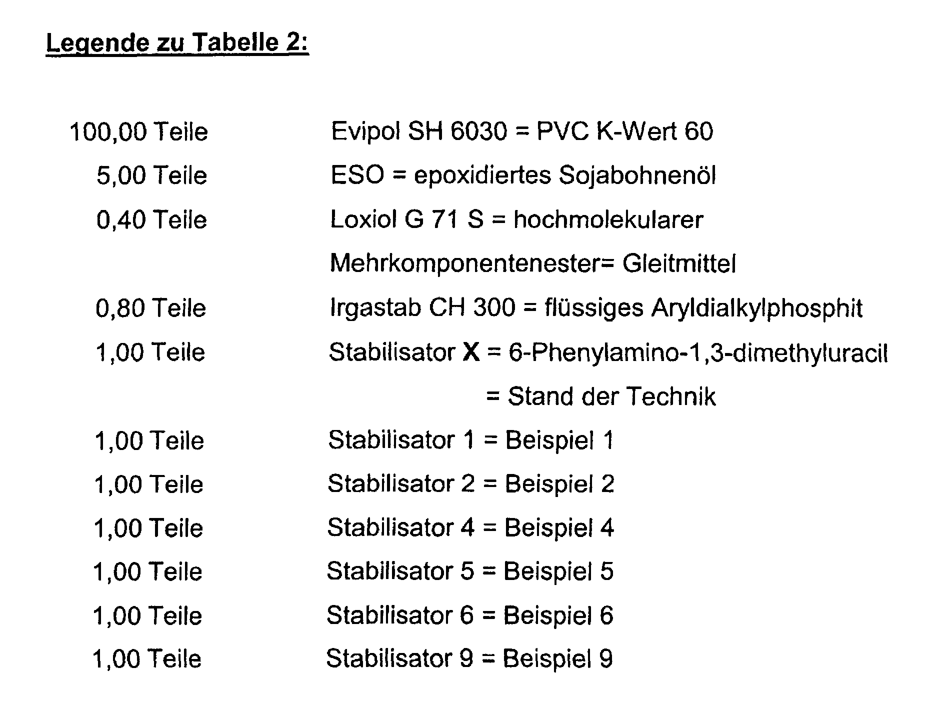

### Beispiel II:

Die Ergebnisse sind der folgenden Tabelle 3 zu entnehmen. Geringe **YI**-Werte bedeuten gute Stabilisierung.

**Tabelle 3**

| Minuten | Stabilisator **X** 0,2 Teile | Stabilisator **1** 0,2 Teile |
|---|---|---|
| 0 | 54,36 | 20,00 |
| 5 | 58,17 | 21,64 |
| 10 | 57,13 | 21,93 |
| 15 | 57,22 | 22,65 |
| 20 | 65,53 | 26,50 |
| 25 | 73,97 | 33,69 |
| 30 | 82,25 | 46,86 |
| 35 | 92,55 | 68,41 |
| 40 | 112,65 | 92,12 |
| 45 | 132,01 | 115,15 |
| 50 | | 131,68 |
| **Legende zu Tabelle 3:** 100,0 Teile Solvic 268 RC = PVC K-Wert 68 3,0 Teile Omyalite 90 T = Kreide 0,6 Teile Ca-stearat 1,0 Teile Hostalub H 4 = modifiziertes Kohlenwasserstoffwachs 0,2 Teile Hostalub H 12 = polares Polyethylenwachs 1,0 Teile Araldit GT 7220 = modifiziertes, festes Bisphenol-A-Epoxidharz 0,4 Teile Irgastab CH 302 = flüssiges Aryl-Alkylphosphit 0,3 Teile Mark 6045 = Gemisch aus 9% NaClO4, 45% CaC03, 40% CaSiO3, 6% H₂O 0,2 Teile Stabilisator **X** = 6-Phenylamino-1,3-dimethyluracil = Stand der Technik 0,2 Teile Stabilisator 1 = Beispiel 1 | | |

Die Beispiele zeigen deutlich, dass die erfindungsgemässen Stabilisatoren sowohl in der Anfangsfarbe und Farbhaltung (Mittelfarbe) - Messwerte **Yl** - als auch in der Langzeitstabilität - Messwert **YI** - verbesserte Ergebnisse gegenüber dem Stand der Technik liefern.

## Patentansprüche

1. Verbindungen der allgemeinen Formel I 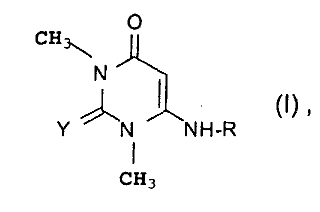 wobei
Y Sauerstoff oder Schwefel ist, und
R mit -OH substituiertes Phenyl oder mit -OH und 1 bis 3 C₁-C₄-Alkyl oder mit C₁-C₄-Alkoxy substituiertes Phenyl darstellt.

2. Verbindungen gemäss Anspruch **1,** wobei
Y Sauerstoff ist.

3. Zusammensetzung enthaltend ein chlorhaltiges Polymer und mindestens eine Verbindung der allgemeinen Formel I gemäss Anspruch **1**.

4. Zusammensetzung gemäss Anspruch **3** enthaltend mindestens einen epoxidierten Fettsäureester.

5. Zusammensetzung gemäss Anspruch **3** enthaltend mindestens ein Zinkund/oder Alkali- und/oder Erdalkalicarboxylat oder Aluminiumcarboxylat.

6. Zusammensetzung gemäss Anspruch **3** enthaltend mindestens einen weiteren Stoff aus den Gruppen der Phosphite, Antioxidantien, beta-Dicarbonylverbindungen, Weichmacher, Füllstoffe, Gleitmittel oder Pigmente.

7. Zusammensetzung gemäss Anspruch **3** enthaltend Kreide als Füllstoff.

8. Zusammensetzung gemäss Anspruch **3** enthaltend Calciumstearat als Gleitmittel.

9. Zusammensetzung gemäss Anspruch **3** enthaltend Titandioxid und/oder Zirkonoxid und/oder Bariumsulfat als Pigment.

10. Zusammensetzung gemäss Anspruch **3** enthaltend mindestens ein Polyol und/oder einen Disaccharidalkohol.

11. Zusammensetzung gemäss Anspruch **3** enthaltend mindestens eine Glycidylverbindung.

12. Zusammensetzung gemäss Anspruch **3** enthaltend mindestens eine Perchloratverbindung.

13. Zusammensetzung gemäss Anspruch **3** enthaltend mindestens eine zeolithische Verbindung.

14. Zusammensetzung gemäss Anspruch **3** enthaltend mindestens eine Schichtgitterverbindung (Hydrotalcite).

15. Zusammensetzung gemäss Anspruch **3** enthaltend mindestens ein Hydrotalcit.

16. Verfahren zur Stabilisierung chlorhaltiger Polymere, **dadurch gekennzeichnet, dass** man diesen mindestens eine Verbindung der Formel I gemäss
Anspruch **1** einverleibt.

17. Verwendung der Verbindungen der allgemeinen Formel I gemäss Anspruch **1** als Stabilisatoren von halogenhaltigen Polymeren.

18. Verwendung der Verbindungen der allgemeinen Formel I gemäss Anspruch **1**
als Stabilisatoren von recyclierten halogenhaltigen Polymeren.

## Claims

1. Compounds of the general formula I 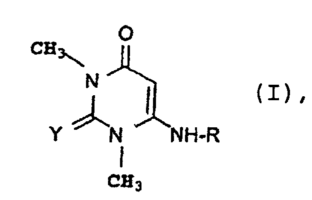 where
Y is oxygen or sulphur, and
R is -OH-substituted phenyl or phenyl which is substituted by -OH and from 1 to 3 times by C₁-C₄-alkyl or C₁-C₄-alkoxy-substituted phenyl.

2. Compounds according to Claim **1,** where
Y is oxygen.

3. Composition comprising a chlorine-containing polymer and at least one compound of the general formula I according to Claim **1.**

4. Composition according to Claim **3** comprising at least one epoxidized fatty acid ester.

5. Composition according to Claim **3** comprising at least one zinc carboxylate and/or alkali metal carboxylate and/or alkaline earth metal carboxylate and/or aluminium carboxylate.

6. Composition according to Claim **3** comprising at least one further substance from the groups of the phosphites, antioxidants, beta-dicarbonyl compounds, plasticizers, fillers, lubricants or pigments.

7. Composition according to Claim **3** comprising chalk as filler.

8. Composition according to Claim **3** comprising calcium stearate as lubricant.

9. Composition according to Claim **3** comprising titanium dioxide and/or zirconium oxide and/or barium sulphate as pigment.

10. Composition according to Claim **3** comprising at least one polyol and/or a disaccharide alcohol.

11. Composition according to Claim **3** comprising at least one glycidyl compound.

12. Composition according to Claim **3** comprising at least one perchlorate compound.

13. Composition according to Claim **3** comprising at least one zeolite compound.

14. Composition according to Claim **3** comprising at least one layered lattice compound (hydrotalcites).

15. Composition according to Claim **3** comprising at least one hydrotalcite.

16. Method of stabilizing chlorine-containing polymers, **characterized in that** at least one compound of the formula I according to Claim **1** is incorporated into said polymers.

17. Use of the compounds of the general formula 1 according to Claim **1** as stabilizers for halogen-containing polymers.

18. Use of the compounds of the general formula 1 according to Claim **1** as stabilizers for recycled halogen-containing polymers.

## Revendications

1. Composés de formule générale I 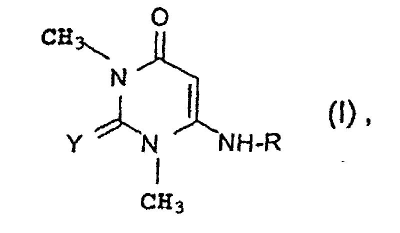 dans laquelle
Y est un oxygène ou un soufre et
R représente un phényle substitué par -OH ou un phényle substitué par -OH et 1 à 3 alkyles en C₁ à C₄ ou un alcoxy en C₁ à C₄.

2. Composés selon la revendication 1, Y étant un oxygène.

3. Composition contenant un polymère contenant du chlore et au moins un composé de formule générale I selon la revendication 1.

4. Composition selon la revendication 3, contenant au moins un ester d'acide gras époxydé.

5. Composition selon la revendication 3, contenant au moins un carboxylate de zinc et/ou alcalin et/ou alcalino-terreux ou un carboxylate d'aluminium.

6. Composition selon la revendication 3, contenant au moins une autre substance des groupes des phosphites, antioxydants, composés bêta-dicarbonyle, plastifiants, charges, lubrifiants ou pigments.

7. Composition selon la revendication 3, contenant de la craie comme charge.

8. Composition selon la revendication 3, contenant du stéarate de calcium comme lubrifiant.

9. Composition selon la revendication 3 contenant du dioxyde de titane et/ou de l'oxyde de zirconium et/ou du sulfate de baryum comme pigment.

10. Composition selon la revendication 3, contenant au moins un polyol et/ou un alcool de disaccharide.

11. Composition selon la revendication 3, contenant au moins un composé glycidyle.

12. Composition selon la revendication 3, contenant au moins un composé perchlorate.

13. Composition selon la revendication 3, contenant au moins un composé zéolithe.

14. Composition selon la revendication 3, contenant au moins un composé mica (hydrotalcites).

15. Composition selon la revendication 3, contenant au moins un hydrotalcite.

16. Procédé pour la stabilisation de polymères contenant du chlore, **caractérisé en ce qu'**on y incorpore au moins un composé de formule I selon la revendication 1.

17. Utilisation des composés de formule générale I selon la revendication 1 comme stabilisateurs de polymères contenant de l'halogène.

18. Utilisation des composés de formule générale I selon la revendication 1 comme stabilisateurs de polymères recyclés contenant de l'halogène.
